# EUROPEAN PATENT SPECIFICATION

(11) **EP 2 332 534 B1**
(45) Date of publication and mention of the grant of the patent: **14.11.2018**
(21) Application number: 10015055.6
(22) Date of filing: 22.02.2007
(51) Int. Cl.: A61K 31/355, A61P 3/00, A61P 25/28, A61K 31/05

(54) **Phenol and benzoquinone derivatives for use in the treatment of mitochondrial diseases and modulation of energy biomarkers**
Phenol- und Benzoquinonederivate zur Verwendung in der Behandlung von Mitochondriopathien und zur Modulation von Energie-Biomarkern
Dérivés du phénol et de la benzoquinone pour leur utilisation dans le traitement des maladies mitochondriales et dans la modulation de biomarqueurs énergétiques

(30) Priority: 22.02.2006 US 776028 P; 06.12.2006 US 873395 P
(43) Date of publication of application: 15.06.2011
(62) Divisional of application: 07751472.7
(73) Proprietor: BIOELECTRON TECHNOLOGY CORPORATION, Mountain View, CA 94043 (US)
(72) Inventor: Miller, Guy, M., Mountain View, CA 94043 (US); Hecht, Sidney, M., Charlottesville, VA 22904-4319 (US); Jankowski, Orion, D., Mountain View, CA 94043 (US); Wesson, Kieron, E., Mountain View, CA 94043 (US); Mollard, Paul, Mountain View, CA 94043 (US)
(74) Representative: J A Kemp

(56) References cited:
- HENDLIN, DAVID ET AL: "Activity of coenzyme Q10 and its analogs in the succinoxidase system of electron transport particles", JOURNAL OF BIOLOGICAL CHEMISTRY , 235, 1187-91 CODEN: JBCHA3; ISSN: 0021-9258, 1960, XP002630274,
- JAUSLIN MATTHIAS L ET AL: "Mitochondria-targeted antioxidants protect Friedreich Ataxia fibroblasts from endogenous oxidative stress more effectively than untargeted antioxidants.", THE FASEB JOURNAL : OFFICIAL PUBLICATION OF THE FEDERATION OF AMERICAN SOCIETIES FOR EXPERIMENTAL BIOLOGY. OCT 2003, vol. 17, no. 13, October 2003 (2003-10), pages 1972-1974, XP002409006, ISSN: 1530-6860
- WALTER L ET AL: "Three classes of ubiquinone analogs regulate the mitochondrial permeability transition pore through a common site", JOURNAL OF BIOLOGICAL CHEMISTRY, AMERICAN SOCIETY FOR BIOCHEMISTRY AND MOLECULAR BIOLOGY, INC, US, vol. 275, no. 38, 22 September 2000 (2000-09-22), pages 29521-29527, XP002474841, ISSN: 0021-9258, DOI: DOI:10.1074/JBC.M004128200
- GILLE L ET AL: "Effects of tocopheryl quinone on the heart: Model experiments with xanthine oxidase, heart mitochondria, and isolated perfused rat hearts", FREE RADICAL BIOLOGY AND MEDICINE, ELSEVIER SCIENCE, US, vol. 30, no. 8, 15 April 2001 (2001-04-15) , pages 865-876, XP002474923, ISSN: 0891-5849, DOI: DOI:10.1016/S0891-5849(01)00475-0
- LIPSHUTZ B H ET AL: "An Expeditious Route to CoQn, Vitamins K1 and K2, and Related Allylated para-Quinones Utilizing Ni(0) Catalysis", TETRAHEDRON, ELSEVIER SCIENCE PUBLISHERS, AMSTERDAM, NL, vol. 54, no. 7, 12 February 1998 (1998-02-12), pages 1241-1253, XP004107470, ISSN: 0040-4020, DOI: DOI:10.1016/S0040-4020(97)10222-8

## Description

### TECHNICAL FIELD

The application discloses compositions useful for treatment or suppression of diseases due to mitochondrial disorders, such as Friedreich's ataxia, Leber's Hereditary Optic Neuropathy, Kearns-Sayre Syndrome, and mitochondrial myopathy, encephalopathy, lactacidosis, stroke, and for modulating energy biomarkers in a subject.

### BACKGROUND

Mitochondria are organelles in eukaryotic cells, popularly referred to as the "powerhouse" of the cell. The molecule adenosine triphosphate (ATP) functions as an energy "currency" or energy carrier in the cell, and eukaryotic cells derive the majority of their ATP from biochemical processes carried out by mitochondria. These biochemical processes include the citric acid cycle (the tricarboxylic acid cycle, or Kreb's cycle), which generates reduced nicotinamide adenine dinucleotide (NADH + H⁺) from oxidized nicotinamide adenine dinucleotide (NAD⁺), and oxidative phosphorylation, during which NADH + H⁺ is oxidized back to NAD⁺. (The citric acid cycle also reduces flavin adenine dinucleotide, or FAD, to FADH₂; FADH₂ also participates in oxidative phosphorylation.)

The electrons released by oxidation of NADH + H⁺ are shuttled down a series of protein complexes (Complex I, Complex II, Complex III, and Complex IV) known as the respiratory chain. These complexes are embedded in the inner membrane of the mitochondrion. Complex IV, at the end of the chain, transfers the electrons to oxygen, which is reduced to water. The energy released as these electrons traverse the complexes is used to generate a proton gradient across the inner membrane of the mitochondrion, which creates an electrochemical potential across the inner membrane. Another protein complex, Complex V (which is not directly associated with Complexes I, II, III and IV) uses the energy stored by the electrochemical gradient to convert ADP into ATP.

The citric acid cycle and oxidative phosphorylation are preceded by glycolysis, in which a molecule of glucose is broken down into two molecules of pyruvate, with net generation of two molecules of ATP per molecule of glucose. The pyruvate molecules then enter the mitochondria, where they are completely oxidized to CO₂ and H₂O via oxidative phosphorylation (the overall process is known as aerobic respiration). The complete oxidation of the two pyruvate molecules to carbon dioxide and water yields about at least 28-29 molecules of ATP, in addition to the 2 molecules of ATP generated by transforming glucose into two pyruvate molecules. If oxygen is not available, the pyruvate molecule does not enter the mitochondria, but rather is converted to lactate, in the process of anaerobic respiration.

The overall net yield per molecule of glucose is thus approximately at least 30-31 ATP molecules. ATP is used to power, directly or indirectly, almost every other biochemical reaction in the cell. Thus, the extra (approximately) at least 28 or 29 molecules of ATP contributed by oxidative phosphorylation during aerobic respiration are critical to the proper functioning of the cell. Lack of oxygen prevents aerobic respiration and will result in eventual death of almost all aerobic organisms; a few organisms, such as yeast, are able to survive using either aerobic or anaerobic respiration.

When cells in an organism are temporarily deprived of oxygen, anaerobic respiration is utilized until oxygen again becomes available or the cell dies. The pyruvate generated during glycolysis is converted to lactate during anaerobic respiration. The buildup of lactic acid is believed to be responsible for muscle fatigue during intense periods of activity, when oxygen cannot be supplied to the muscle cells. When oxygen again becomes available, the lactate is converted back into pyruvate for use in oxidative phosphorylation.

Genetic defects in the proteins making up the respiratory chain lead to severe disease states. One such disease is Friedreich's ataxia (FRDA or FA). Friedreich's ataxia is an autosomal recessive neurodegenerative and cardiodegenerative disorder caused by decreased levels of the protein frataxin. Frataxin is important for the assembly of iron-sulfur clusters in mitochondrial respiratory-chain complexes. Estimates of the prevalence of FRDA in the United States range from 1 in every 22,000-29,000 people (see World-Wide-Web address .nlm.nih.gov/medlineplus/ency/article/001411.htm) to 1 in 50,000 people (World-Wide-Web address .umc-cares.org/health_info/ADAM/Articles/001411.asp). The disease causes the progressive loss of voluntary motor coordination (ataxia) and cardiac complications. Symptoms typically begin in childhood, and the disease progressively worsens as the patient grows older; patients eventually become wheelchair-bound due to motor disabilities.

Another disease linked to mitochondrial dysfunction is Leber's Hereditary Optic Neuropathy (LHON). The disease is characterized by blindness which occurs on average between 27 and 34 years of age (World-Wide-Web address .ncbi.nlm.nih.gov/entrez/dispomim.cgi?id=535000); blindness can develop in both eyes simultaneously, or sequentially (one eye will develop blindness, followed by the other eye two months later on average). Other symptoms may also occur, such as cardiac abnormalities and neurological complications.

Yet another devastating syndrome resulting from mitochondrial defects is mitochondrial myopathy, encephalopathy, lactacidosis, and stroke (MELAS). The disease can manifest itself in infants, children, or young adults. Strokes, accompanied by vomiting and seizures, are one of the most serious symptoms; it is postulated that the metabolic impairment of mitochondria in certain areas of the brain is responsible for cell death and neurological lesions, rather than the impairment of blood flow as occurs in ischemic stroke. Other severe complications, including neurological symptoms, are often present, and elevated levels of lactic acid in the blood occur.

Another mitochondrial disease is Kearns-Sayre Syndrome (KSS). KSS is characterized by a triad of features including: (1) typical onset in persons younger than age 20 years; (2) chronic, progressive, external ophthalmoplegia; and (3) pigmentary degeneration of the retina. In addition, KSS may include cardiac conduction defects, cerebellar ataxia, and raised cerebrospinal fluid (CSF) protein levels (e.g., >100 mg/dL). Additional features associated with KSS may include myopathy, dystonia, endocrine abnormalities (e.g., diabetes, growth retardation or short stature, and hypoparathyroidism), bilateral sensorineural deafness, dementia, cataracts, and proximal renal tubular acidosis. Thus, KSS may affect many organ systems.

The four diseases above appear to be caused by defects in complex I of the respiratory chain. Electron transfer from complex I to the remainder of the respiratory chain is mediated by the compound coenzyme Q (also known as ubiquinone). Oxidized coenzyme Q (CoQ^{ox} or ubiquinone) is reduced by complex I to reduced coenzyme Q (CoQ^{red} or ubiquinol). The reduced coenzyme Q then transfers its electrons to complex III of the respiratory chain (skipping over complex II), where it is re-oxidized to CoQ^{ox} (ubiquinone). CoQ^{ox} can then participate in further iterations of electron transfer.

*In vitro* mitochondrial activity of CoQ and analogues thereof is disclosed in the following article "the activity of coenzyme Q10 and its analogues in the succinoxidase system of electron transport particles" in the Journal of Biological Chemistry, 1960, vol. 235, pages 1187-1191.

Very few treatments are available for patients suffering from these diseases. Recently, the compound idebenone has been proposed for treatment of Friedreich's ataxia. While the clinical effects of idebenone have been relatively modest, the complications of mitochondrial diseases can be so severe that even marginally useful therapies are preferable to the untreated course of the disease. Another compound, MitoQ, has been proposed for treating mitochondrial disorders (see U.S. Patent Application Publication No. 2005/0043553); clinical results for MitoQ have not yet been reported. For KSS, administration of coenzyme Q10 (CoQ10) and vitamin supplements have shown only transient beneficial effects in individual cases.

Accordingly, there is a serious and unmet need for effective treatments of mitochondrial disorders, such as Friedreich's ataxia, Leber's hereditary optic neuropathy, MELAS, and Kearns-Sayre Syndrome.

The ability to adjust biological production of energy has applications beyond the diseases described above. Various other disorders can result in suboptimal levels of energy biomarkers (sometimes also referred to as indicators of energetic function), such as ATP levels. Treatments for these disorders are also needed, in order to modulate one or more energy biomarkers to improve the health of the patient. In other applications, it can be desirable to modulate certain energy biomarkers away from their normal values in an individual that is not suffering from disease. For example, if an individual is undergoing an extremely strenuous undertaking, it can be desirable to raise the level of ATP in that individual.

### DISCLOSURE OF THE INVENTION

The invention embraces compounds as described herein, which are useful for treating a mitochondrial disorder and the disclosure embraces compounds as described herein which are useful for modulating one or more energy biomarkers, normalizing one or more energy biomarkers, or enhancing one or more energy biomarkers.

In one embodiment, the invention embraces a compound for use in a method of treating a mitochondrial disorder, wherein the compound is of the formula: 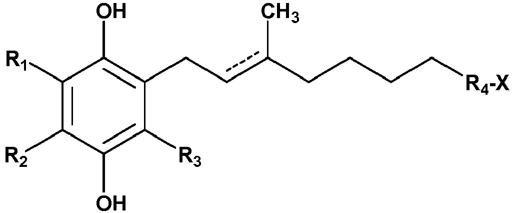 or 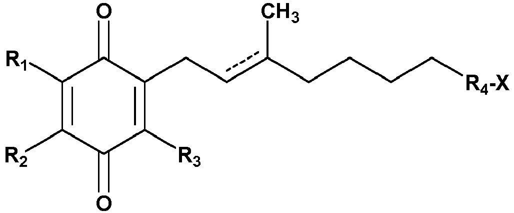
wherein the bond indicated with a dashed line can be single or double;
where R₁, R₂, and R₃ are independently selected from the group consisting of -H and -C₁-C₅ alkyl;
where R₄ represents a linear or branched group containing 1 to 32 carbon atoms and any number of single, double, or triple bonds in any chemically possible combination; where X is -H;
where alkyl refers to a straight-chain, branched-chain, or cyclic saturated aliphatic group, and combinations thereof, having the number of carbon atoms specified;
wherein the cyclic group consists of one ring or multiple fused rings;
or any stereoisomer, mixture of stereoisomers, or salt thereof.

R₁, R₂, and R₃ are independently selected from the group consisting of -H and -C₁-C₅ alkyl. In another embodiment, at least one of R₁, R₂, and R₃ is independently selected from -C₂-C₅ alkyl. In another embodiment, at least two of R₁, R₂, and R₃ are independently selected from -C₂-C₅ alkyl. In another embodiment, R₁ and R₂ are -CH₃, R₄ is -CH₂CH₂-, and X is -H. In another embodiment, the one or more compounds are selected from compounds of the formula: 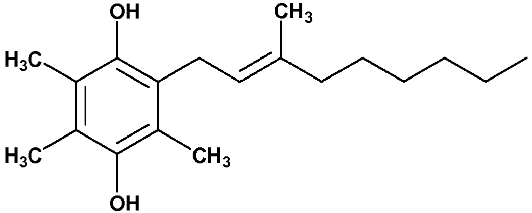 or 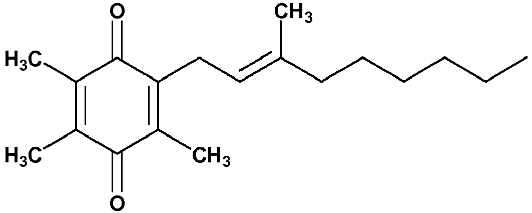 or 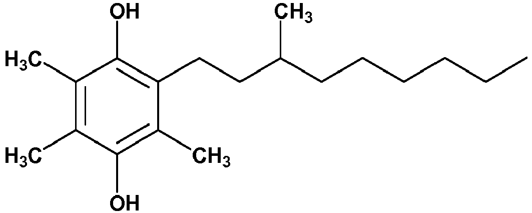 or 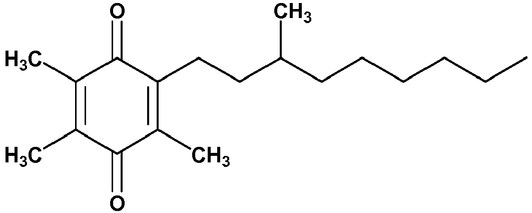 or 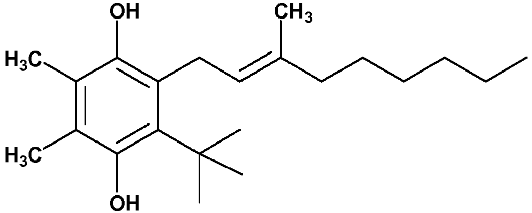 or 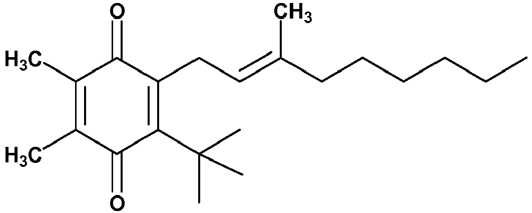 or any stereoisomer, mixture of stereoisomers thereof.

In another embodiment, R₄ is -(CH₂)ₙC(CH₃)₂-, where n is an integer from 0 to 15 inclusive.

In another embodiment, at least one of R₁, R₂, and R₃ is independently selected from -C₁-C₅ alkyl. In another embodiment, at least two of R₁, R₂, and R₃ are independently selected from -C₁-C₅ alkyl. In another embodiment, R₁, R₂, and R₃ are independently selected from -C₁-C₅ alkyl.

In any of the methods above, the compound or compounds to be administered can be combined with a pharmaceutically acceptable excipient.

In any of the methods above, the mitochondrial disorder can be selected from the group consisting of inherited mitochondrial diseases; Myoclonic Epilepsy with Ragged Red Fibers (MERRF); Mitochondrial Myopathy, Encephalopathy, Lactacidosis, Stroke (MELAS); Leber's Hereditary Optic Neuropathy (LHON); Leigh Syndrome; Kearns-Sayre Syndrome (KSS); Friedreich's Ataxia (FA); myopathies; cardiomyopathy; encephalomyopathy; renal tubular acidosis; neurodegenerative diseases; Parkinson's disease; Alzheimer's disease; amyotrophic lateral sclerosis (ALS); motor neuron diseases; neurological diseases; epilepsy; genetic diseases; Huntington's Disease; mood disorders; schizophrenia; bipolar disorder; age-associated diseases; macular degeneration; diabetes; and cancer. In another embodiment, the mitochondrial disorder can be selected from the group consisting of inherited mitochondrial diseases; Myoclonic Epilepsy with Ragged Red Fibers (MERRF); Mitochondrial Myopathy, Encephalopathy, Lactacidosis, Stroke (MELAS); Leber's Hereditary Optic Neuropathy (LHON); Leigh Syndrome; Kearns-Sayre Syndrome (KSS); and Friedreich's Ataxia (FA).

In any of the methods above for modulating one or more energy biomarkers, normalizing one or more energy biomarkers, or enhancing one or more energy biomarkers, the energy biomarker can be selected from the group consisting of: lactic acid (lactate) levels, either in whole blood, plasma, cerebrospinal fluid, or cerebral ventricular fluid; pyruvic acid (pyruvate) levels, either in whole blood, plasma, cerebrospinal fluid, or cerebral ventricular fluid; lactate/pyruvate ratios, either in whole blood, plasma, cerebrospinal fluid, or cerebral ventricular fluid; phosphocreatine levels, NADH (NADH +H⁺) levels; NADPH (NADPH+H⁺) levels; NAD levels; NADP levels; ATP levels; reduced coenzyme Q (CoQ^{red}) levels; oxidized coenzyme Q (CoQ^{ox}) levels; total coenzyme Q (CoQ^{tot}) levels; oxidized cytochrome C levels; reduced cytochrome C levels; oxidized cytochrome C/reduced cytochrome C ratio; acetoacetate levels, β-hydroxy butyrate levels, acetoacetate/β-hydroxy butyrate ratio, 8-hydroxy-2'-deoxyguanosine (8-OHdG) levels; levels of reactive oxygen species; levels of oxygen consumption (VO2); levels of carbon dioxide output (VCO2); respiratory quotient (VCO2/VO2); exercise tolerance; and anaerobic threshold.

In any of the above methods, the subject can be selected from the group consisting of: a subject with a mitochondrial disease; a subject undergoing strenuous or prolonged physical activity; a subject with chronic energy problems; a subject with chronic respiratory problems; a pregnant female; a pregnant female in labor; a neonate; a premature neonate; a subject exposed to an extreme environment; a subject exposed to a hot environment; a subject exposed to a cold environment; a subject exposed to an environment with lower-than-average oxygen content; a subject exposed to an environment with higher-than-average carbon dioxide content; a subject exposed to an environment with higher-than-average levels of air pollution; a subject with lung disease; a subject with lower-than-average lung capacity; a tubercular patient; a lung cancer patient; an emphysema patient; a cystic fibrosis patient; a subject recovering from surgery; a subject recovering from illness; a subject undergoing acute trauma; a subject in shock; a subject requiring acute oxygen administration; a subject requiring chronic oxygen administration; an elderly subject; an elderly subject experiencing decreased energy; and a subject suffering from chronic fatigue.

In another embodiment, the invention embraces compounds of the formula: 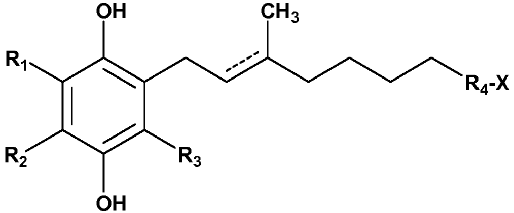 or 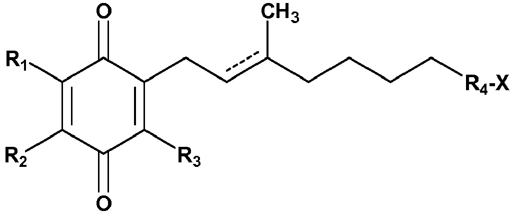
wherein the bond indicated with a dashed line can be single or double;
where R₁, R₂, and R₃ are independently selected from the group consisting of -H and -C₁-C₅ alkyl, where at least one of R₁, R₂, and R₃ is independently selected from - C₂-C₅ alkyl;
where R₄ represents a linear or branched group containing 1 to 32 carbon atoms and any number of single, double, or triple bonds in any chemically possible combination; where X is -H;
where alkyl refers to a straight-chain, branched chain, or cyclic saturated aliphatic group, and combinations thereof, having the number of carbon atoms specified; wherein the cyclic group consists of one ring or multiple fused rings;
or any stereoisomer, mixture of stereoisomers, or salt thereof.

In another embodiment, at least two of R₁, R₂, and R₃ are independently selected from -C₂-C₅ alkyl. In another embodiment, R₁, R₂, and R₃ are independently selected from -C₂-C₅ alkyl.

In another embodiment, the invention embraces compounds of the formula: 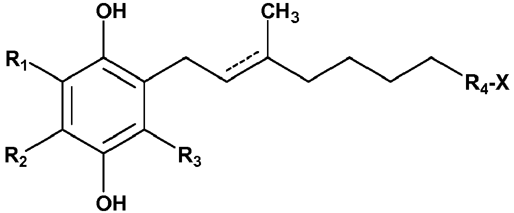 or 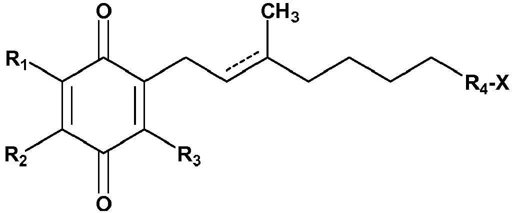
wherein the bond indicated with a dashed line can be single or double;
where R₁, R₂, and R₃ are independently selected from the group consisting of -H and -C₁-C₅ alkyl;
where R₄ is -(CH₂)ₙC(CH₃)₂-, where n is an integer from 0 to 15 inclusive;
where X is -H;
where alkyl refers to a straight-chain, branched-chain, or cyclic saturated aliphatic group, and combinations thereof, having the number of carbon atoms specified; wherein the cyclic group consists of one ring or multiple fused rings;
or any stereoisomer, mixture of stereoisomers, or salt thereof.

For any of the compounds described above, the compound can be combined with a pharmaceutically acceptable excipient.

In another embodiment, the invention embraces compounds for use in a method of treating or suppressing a mitochondrial disorder and the disclosure embraces compounds for use in a method of modulating one or more energy biomarkers, normalizing one or more energy biomarkers, or enhancing one or more energy biomarkers.

In another embodiment, the invention embraces compounds for use in a method of treating or suppressing a mitochondrial disorder and the disclosure embraces compounds for use in a method of modulating one or more energy biomarkers, normalizing one or more energy biomarkers, or enhancing one or more energy biomarkers.

In other embodiments, including any of the foregoing embodiments, the mitochondrial disorder is selected from the group consisting of inherited mitochondrial diseases; Myoclonic Epilepsy with Ragged Red Fibers (MERRF); Mitochondrial Myopathy, Encephalopathy, Lactacidosis, Stroke (MELAS); Leber's Hereditary Optic Neuropathy (LHON); Leigh Disease; Kearns-Sayre Syndrome (KSS); Friedreich's Ataxia (FA); other myopathies; cardiomyopathy; encephalomyopathy; renal tubular acidosis; neurodegenerative diseases; Parkinson's disease; Alzheimer's disease; amyotrophic lateral sclerosis (ALS); motor neuron diseases; other neurological diseases; epilepsy; genetic diseases; Huntington's Disease; mood disorders; schizophrenia; bipolar disorder; age-associated diseases; macular degeneration; diabetes; and cancer.

In another embodiment, including any of the foregoing embodiments, the mitochondrial disorder is selected from the group consisting of inherited mitochondrial diseases; Myoclonic Epilepsy with Ragged Red Fibers (MERRF); Mitochondrial Myopathy, Encephalopathy, Lactacidosis, Stroke (MELAS); Leber's Hereditary Optic Neuropathy (LHON); Leigh Disease; Kearns-Sayre Syndrome (KSS); and Friedreich's Ataxia (FA).

In another embodiment of the invention, including any of the foregoing embodiments, the mitochondrial disorder is Friedreich's ataxia (FRDA). In another embodiment of the invention, the mitochondrial disorder is Leber's Hereditary Optic Neuropathy (LHON). In another embodiment of the invention, the mitochondrial disorder is mitochondrial myopathy, encephalopathy, lactacidosis, stroke (MELAS). In another embodiment of the invention, the mitochondrial disorder is Kearns-Sayre Syndrome (KSS). In another embodiment of the invention, the mitochondrial disorder is Myoclonic Epilepsy with Ragged Red Fibers (MERRF). In another embodiment of the invention, the mitochondrial disorder is Parkinson's disease.

In another embodiment of the disclosure including any of the foregoing embodiments, the compounds described herein are used in a method of treating a mitochondrial disorder to modulate one or more of various energy biomarkers, including, but not limited to, lactic acid (lactate) levels, either in whole blood, plasma, cerebrospinal fluid, or cerebral ventricular fluid; pyruvic acid (pyruvate) levels, either in whole blood, plasma, cerebrospinal fluid, or cerebral ventricular fluid; lactate/pyruvate ratios, either in whole blood, plasma, cerebrospinal fluid, or cerebral ventricular fluid; phosphocreatine levels, NADH (NADH +H⁺) or NADPH (NADPH+H⁺) levels; NAD or NADP levels; ATP levels; reduced coenzyme Q (CoQ^{red}) levels; oxidized coenzyme Q (CoQ^{ox}) levels; total coenzyme Q (CoQ^{tot}) levels; oxidized cytochrome C levels; reduced cytochrome C levels; oxidized cytochrome C/reduced cytochrome C ratio; acetoacetate levels; beta-hydroxy butyrate levels; acetoacetate/beta-hydroxy butyrate ratio; 8-hydroxy-2'-deoxyguanosine (8-OHdG) levels; levels of reactive oxygen species; oxygen consumption (VO2), carbon dioxide output (VCO2), respiratory quotient (VCO2/VO2), and to modulate exercise intolerance (or conversely, modulate exercise tolerance) and to modulate anaerobic threshold. Energy biomarkers can be measured in whole blood, plasma, cerebrospinal fluid, cerebroventricular fluid, arterial blood, venous blood, or any other body fluid, body gas, or other biological sample useful for such measurement. In one embodiment, the levels are modulated to a value within about 2 standard deviations of the value in a healthy subject. In another embodiment, the levels are modulated to a value within about 1 standard deviation of the value in a healthy subject. In another embodiment, the levels in a subject are changed by at least about 10% above or below the level in the subject prior to modulation. In another embodiment, the levels are changed by at least about 20% above or below the level in the subject prior to modulation. In another embodiment, the levels are changed by at least about 30% above or below the level in the subject prior to modulation. In another embodiment, the levels are changed by at least about 40% above or below the level in the subject prior to modulation. In another embodiment, the levels are changed by at least about 50% above or below the level in the subject prior to modulation. In another embodiment, the levels are changed by at least about 75% above or below the level in the subject prior to modulation. In another embodiment, the levels are changed by at least about 100% above or at least about 90% below the level in the subject prior to modulation.

In another embodiment, including any of the foregoing embodiments, the subject or subjects in which compounds are used in a method of treating or suppressing a mitochondrial disorder, modulating one or more energy biomarkers, normalizing one or more energy biomarkers, or enhancing one or more energy biomarkers is performed is/are selected from the group consisting of subjects undergoing strenuous or prolonged physical activity; subjects with chronic energy problems; subjects with chronic respiratory problems; pregnant females; pregnant females in labor; neonates; premature neonates; subjects exposed to extreme environments; subjects exposed to hot environments; subjects exposed to cold environments; subjects exposed to environments with lower-than-average oxygen content; subjects exposed to environments with higher-than-average carbon dioxide content; subjects exposed to environments with higher-than-average levels of air pollution; airline travelers; flight attendants; subjects at elevated altitudes; subjects living in cities with lower-than-average air quality; subjects working in enclosed environments where air quality is degraded; subjects with lung diseases; subjects with lower-than-average lung capacity; tubercular patients; lung cancer patients; emphysema patients; cystic fibrosis patients; subjects recovering from surgery; subjects recovering from illness; elderly subjects; elderly subjects experiencing decreased energy; subjects suffering from chronic fatigue; subjects suffering from chronic fatigue syndrome; subjects undergoing acute trauma; subjects in shock; subjects requiring acute oxygen administration; subjects requiring chronic oxygen administration; or other subjects with acute, chronic, or ongoing energy demands who can benefit from enhancement of energy biomarkers.

In another embodiment, the invention embraces one or more compounds described herein in combination with a pharmaceutically acceptable excipient, carrier, or vehicle.

In another embodiment, the invention embraces one or more compounds described herein for use in therapy. In another embodiment, the invention embraces one or more compounds described herein for use in the therapy of mitochondrial disease. In another embodiment, the invention embraces one or more compounds described herein in the manufacture of a medicament for use in therapy of mitochondrial disease.

For all of the compounds and methods described above, the quinone form can also be used in its reduced (hydroquinone) form when desired. Likewise, the hydroquinone form can also be used in its oxidized (quinone) form when desired.

For all of the compounds and methods described above, R₁, R₂, and R₃, when present, can be selected from the group consisting of H and C₁-C₅ alkyl, or from C₁-C₅ alkyl.

### MODES FOR CARRYING OUT THE INVENTION

The invention embraces compounds as defined above useful in treating or suppressing mitochondrial disorders, and the disclosure embraces compounds for use in methods for modulation of energy biomarkers. The redox active therapeutics for treatment or suppression of mitochondrial diseases and associated aspects of the invention are described in more detail herein.

By "subject," "individual," or "patient" is meant an individual organism, preferably a vertebrate, more preferably a mammal, most preferably a human.

"Treating" a disease with the compounds and methods discussed herein is defined as administering one or more of the compounds discussed herein, with or without additional therapeutic agents, in order to reduce or eliminate either the disease or one or more symptoms of the disease, or to retard the progression of the disease or of one or more symptoms of the disease, or to reduce the severity of the disease or of one or more symptoms of the disease. "Suppression" of a disease with the compounds and methods discussed herein is defined as administering one or more of the compounds discussed herein, with or without additional therapeutic agents, in order to suppress the clinical manifestation of the disease, or to suppress the manifestation of adverse symptoms of the disease. The distinction between treatment and suppression is that treatment occurs after adverse symptoms of the disease are manifest in a subject, while suppression occurs before adverse symptoms of the disease are manifest in a subject. Suppression may be partial, substantially total, or total. Because many of the mitochondrial disorders are inherited, genetic screening can be used to identify patients at risk of the disease. The compounds and methods of the invention can then be administered to asymptomatic patients at risk of developing the clinical symptoms of the disease, in order to suppress the appearance of any adverse symptoms. "Therapeutic use" of the compounds discussed herein is defined as using one or more of the compounds discussed herein to treat or suppress a disease, as defined above. An "effective amount" of a compound is an amount of the compound sufficient to modulate, normalize, or enhance one or more energy biomarkers (where modulation, normalization, and enhancement are defined below). A "therapeutically effective amount" of a compound is an amount of the compound, which, when administered to a subject, is sufficient to reduce or eliminate either a disease or one or more symptoms of a disease, or to retard the progression of a disease or of one or more symptoms of a disease, or to reduce the severity of a disease or of one or more symptoms of a disease, or to suppress the clinical manifestation of a disease, or to suppress the manifestation of adverse symptoms of a disease. A therapeutically effective amount can be given in one or more administrations. An "effective amount" of a compound embraces both a therapeutically effective amount, as well as an amount effective to modulate, normalize, or enhance one or more energy biomarkers in a subject.

"Modulation" of, or to "modulate," an energy biomarker means to change the level of the energy biomarker towards a desired value, or to change the level of the energy biomarker in a desired direction (e.g., increase or decrease). Modulation can include, but is not limited to, normalization and enhancement as defined below.

"Normalization" of, or to "normalize," an energy biomarker is defined as changing the level of the energy biomarker from a pathological value towards a normal value, where the normal value of the energy biomarker can be 1) the level of the energy biomarker in a healthy person or subject, or 2) a level of the energy biomarker that alleviates one or more undesirable symptoms in the person or subject. That is, to normalize an energy biomarker which is depressed in a disease state means to increase the level of the energy biomarker towards the normal (healthy) value or towards a value which alleviates an undesirable symptom; to normalize an energy biomarker which is elevated in a disease state means to decrease the level of the energy biomarker towards the normal (healthy) value or towards a value which alleviates an undesirable symptom.

"Enhancement" of, or to "enhance," energy biomarkers means to intentionally change the level of one or more energy biomarkers away from either the normal value, or the value before enhancement, in order to achieve a beneficial or desired effect. For example, in a situation where significant energy demands are placed on a subject, it may be desirable to increase the level of ATP in that subject to a level above the normal level of ATP in that subject. Enhancement can also be of beneficial effect in a subject suffering from a disease or pathology such as a mitochondrial disease, in that normalizing an energy biomarker may not achieve the optimum outcome for the subject; in such cases, enhancement of one or more energy biomarkers can be beneficial, for example, higher-than-normal levels of ATP, or lower-than-normal levels of lactic acid (lactate) can be beneficial to such a subject.

By modulating, normalizing, or enhancing the energy biomarker Coenzyme Q is meant modulating, normalizing, or enhancing the variant or variants of Coenzyme Q which is predominant in the species of interest. For example, the variant of Coenzyme Q which predominates in humans is Coenzyme Q10. If a species or subject has more than one variant of Coenzyme Q present in significant amounts (i.e., present in amounts which, when modulated, normalized, or enhanced, can have a beneficial effect on the species or subject), modulating, normalizing, or enhancing Coenzyme Q can refer to modulating, normalizing or enhancing any or all variants of Coenzyme Q present in the species or subject.

While the compounds described herein can occur and can be used as the neutral (non-salt) compound, the description is intended to embrace all salts of the compounds described herein. In one embodiment, the salts of the compounds comprise pharmaceutically acceptable salts. Pharmaceutically acceptable salts are those salts which can be administered as drugs or pharmaceuticals to humans and/or animals and which, upon administration, retain at least some of the biological activity of the free compound (neutral compound or non-salt compound). The desired salt of a basic compound may be prepared by methods known to those of skill in the art by treating the compound with an acid. Examples of inorganic acids include, but are not limited to, hydrochloric acid, hydrobromic acid, sulfuric acid, nitric acid, and phosphoric acid. Examples of organic acids include, but are not limited to, formic acid, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, sulfonic acids, and salicylic acid. Salts of basic compounds with amino acids, such as aspartate salts and glutamate salts, can also be prepared. The desired salt of an acidic compound can be prepared by methods known to those of skill in the art by treating the compound with a base. Examples of inorganic salts of acid compounds include, but are not limited to, alkali metal and alkaline earth salts, such as sodium salts, potassium salts, magnesium salts, and calcium salts; ammonium salts; and aluminum salts. Examples of organic salts of acid compounds include, but are not limited to, procaine, dibenzylamine, N-ethylpiperidine, N,N'-dibenzylethylenediamine, and triethylamine salts. Salts of acidic compounds with amino acids, such as lysine salts, can also be prepared.

The invention also includes all stereoisomers and geometric isomers of the compounds, including diastereomers, enantiomers, and cis/trans (E/Z) isomers. The invention also includes mixtures of stereoisomers and/or geometric isomers in any ratio, including, but not limited to, racemic mixtures.

The term "alkyl" refers to saturated aliphatic groups including straight-chain, branched-chain, cyclic groups, and combinations thereof, having the number of carbon atoms specified, or if no number is specified, having up to 12 carbon atoms. "Straight-chain alkyl" or "linear alkyl" groups refers to alkyl groups that are neither cyclic nor branched, commonly designated as "n-alkyl" groups. Examples of alkyl groups include, but are not limited to, groups such as methyl, ethyl, n-propyl, isopropyl, butyl, n-butyl, isobutyl, sec-butyl, t-butyl, pentyl, n-pentyl, hexyl, heptyl, octyl, nonyl, decyl, undecyl, dodecyl, neopentyl, cyclopropyl, cyclobutyl, cyclopentyl, cyclohexyl, and adamantyl. Cycloalkyl groups can consist of one ring, including, but not limited to, groups such as cycloheptyl, or multiple fused rings, including, but not limited to, groups such as adamantyl or norbornyl. One preferred subset of alkyl groups is C₁-C₅ alkyl, which is intended to embrace methyl (Me), ethyl (Et), propyl (Pr), n-propyl (nPr), isopropyl (iPr), butyl (Bu), n-butyl (nBu), isobutyl (iBu), sec-butyl (sBu), t-butyl (tBu), cyclopropyl (cyclPr), cyclobutyl (cyclBu), cyclopropyl-methyl (cyclPr-Me), methyl-cyclopropane (Me-cyclPr), pentyl, n-pentyl, isopentyl, neopentyl, sec-pentyl, t-pentyl, 1,2-dimethylpropyl, cyclopentyl, and any other alkyl group containing between one and five carbon atoms, where the C₁-C₅ alkyl groups can be attached via any valence on the C₁-C₅ alkyl groups.

Note that "Co alkyl," when it appears, is intended to mean either a non-existent group, or a hydrogen, which will be understood by the context in which it appears. When a Co alkyl group appears as the terminal group on a chain, as for example in -(C=O)-C₀ alkyl, it is intended as a hydrogen atom; thus, -(C=O)-C₀ alkyl is intended to represent -(C=O)-H (an aldehyde). When a Co alkyl group appears between two other groups, as, for example, in -(C=O)-C₀ alkyl-C₆-C₁₀ aryl, it is intended to be a nonentity, thus -(C=O)-C₀ alkyl-C₆-C₁₀ aryl represents -(C=O)-C₆-C₁₀ aryl.

"Substituted alkyl" refers to alkyl groups substituted with one or more substituents including, but not limited to, groups such as halogen (fluoro, chloro, bromo, and iodo), alkoxy, acyloxy, amino, hydroxyl, mercapto, carboxy, benzyloxy, phenyl, benzyl, cyano, nitro, thioalkoxy, carboxaldehyde, carboalkoxy and carboxamide, or a functionality that can be suitably blocked, if necessary for purposes of the invention, with a protecting group. Examples of substituted alkyl groups include, but are not limited to, groups such as -CH₂-OH; -CH₂CH₂CH(NH₂)CH₃, etc. The substituent(s) on the substituted alkyl group may be at any available location on the group. Substituted alkyl embraces the preferred subset of C₁-C₅ haloalkyl, which is intended to embrace any C₁-C₅ alkyl substituent having at least one halogen substituent; the halogen can be attached via any available valence on the C₁-C₅ alkyl group. One further subset of C₁-C₅ haloalkyl is -CF₃, -CCl₃, -CBr₃, and -CI₃. Another further subset of C₁-C₅ haloalkyl is the subset with exactly one halogen substituent. Another further subset of C₁-C₅ haloalkyl is the subset with exactly one chloro substituent. Another further subset of C₁-C₅ haloalkyl is the subset with exactly one fluoro substituent. Another further subset of C₁-C₅ haloalkyl is the subset of C₁-C₅ perhaloalkyl; that is, C₁-C₅ alkyl with all available valences replaced by halogens. Another further subset of C₁-C₅ haloalkyl is the subset of C₁-C₅ perfluoroalkyl; that is, C₁-C₅ alkyl with all available valences replaced by fluorines, such as -CF₃ and -CF₂-CF₃. Another further subset of C₁-C₅ haloalkyl is the subset of C₁-C₅ perchloroalkyl; that is, C₁-C₅ alkyl with all available valences replaced by chlorines.

The term "alkenyl" refers to unsaturated aliphatic groups including straight-chain (linear), branched-chain, cyclic groups, and combinations thereof, having the number of carbon atoms specified, or if no number is specified, having up to 12 carbon atoms, which contain at least one double bond (-C=C-). All double bonds may be independently either (*E*) or (*Z*) geometry, as well as arbitrary mixutures thereof. Examples of alkenyl groups include, but are not limited to, -CH₂-CH=CH-CH₃; and -CH₂-CH₂-cyclohexenyl, where the ethyl group can be attached to the cyclohexenyl moiety at any available carbon valence.

"Haloalkenyl" embraces any C₁-C₅ alkenyl substituent having at least one halogen substituent; the halogen can be attached via any available valence on the C₁-C₅ alkenyl group. One further subset of C₁-C₅ haloalkenyl is the subset with exactly one halogen substituent. Another further subset of C₁-C₅ haloalkenyl is the subset with exactly one chloro substituent. Another further subset of C₁-C₅ haloalkenyl is the subset with exactly one fluoro substituent. Another further subset of C₁-C₅ haloalkenyl is the subset of C₁-C₅ perhaloalkenyl; that is, C₁-C₅ alkenyl with all available valences replaced by halogens. Another further subset of C₁-C₅ haloalkenyl is the subset of C₁-C₅ perfluoroalkenyl; that is, C₁-C₅ alkenyl with all available valences replaced by fluorines. Another further subset of C₁-C₅ haloalkenyl is the subset of C₁-C₅ perchloroalkenyl; that is, C₁-C₅ alkenyl with all available valences replaced by chlorines.

The term "alkynyl" refers to unsaturated aliphatic groups including straight-chain (linear), branched-chain, cyclic groups, and combinations thereof, having the number of carbon atoms specified, or if no number is specified, having up to 12 carbon atoms, which contain at least one triple bond (-C≡C-). "Hydrocarbon chain" or "hydrocarbyl" refers to any combination of straight-chain, branched-chain, or cyclic alkyl, alkenyl, or alkynyl groups, and any combination thereof. "Substituted alkenyl," "substituted alkynyl," and "substituted hydrocarbon chain" or "substituted hydrocarbyl" refer to the respective group substituted with one or more substituents, including, but not limited to, groups such as halogen, alkoxy, acyloxy, amino, hydroxyl, mercapto, carboxy, benzyloxy, phenyl, benzyl, cyano, nitro, thioalkoxy, carboxaldehyde, carboalkoxy and carboxamide, or a functionality that can be suitably blocked, if necessary for purposes of the invention, with a protecting group.

"Haloalkynyl" embraces any C₁-C₅ alkynyl substituent having at least one halogen substituent; the halogen can be attached via any available valence on the C₁-C₅ alkynyl group. One further subset of C₁-C₅ haloalkynyl is the subset with exactly one halogen substituent. Another further subset of C₁-C₅ haloalkynyl is the subset with exactly one chloro substituent. Another further subset of C₁-C₅ haloalkynyl is the subset with exactly one fluoro substituent. Another further subset of C₁-C₅ haloalkynyl is the subset of C₁-C₅ perhaloalkynyl; that is, C₁-C₅ alkynyl with all available valences replaced by halogens. Another further subset of C₁-C₅ haloalkynyl is the subset of C₁-C₅ perfluoroalkynyl; that is, C₁-C₅ alkynyl with all available valences replaced by fluorines. Another further subset of C₁-C₅ haloalkynyl is the subset of C₁-C₅ perchloroalkynyl; that is, C₁-C₅ alkynyl with all available valences replaced by chlorines.

"Aryl" or "Ar" refers to an aromatic group having a single ring (including, but not limited to, groups such as phenyl) or two or more condensed rings (including, but not limited to, groups such as naphthyl or anthryl), and includes both unsubstituted and substituted aryl groups. Aryls, unless otherwise specified, contain from 6 to 12 carbon atoms in the ring portion. A preferred range for aryls is from 6 to 10 carbon atoms in the ring portion. "Substituted aryls" refers to aryls substituted with one or more substituents, including, but not limited to, groups such as alkyl, alkenyl, alkynyl, hydrocarbon chains, halogen, alkoxy, acyloxy, amino, hydroxyl, mercapto, carboxy, benzyloxy, phenyl, benzyl, cyano, nitro, thioalkoxy, carboxaldehyde, carboalkoxy and carboxamide, or a functionality that can be suitably blocked, if necessary for purposes of the invention, with a protecting group. "Aralkyl" designates an alkyl-substituted aryl group, where any aryl can attached to the alkyl; the alkyl portion is a straight or branched chain of 1 to 6 carbon atoms, preferably the alkyl chain contains 1 to 3 carbon atoms. When an aralkyl group is indicated as a substituent, the aralkyl group can be connected to the remainder of the molecule at any available valence on either its alkyl moiety or aryl moiety; e.g., the tolyl aralkyl group can be connected to the remainder of the molecule by replacing any of the five hydrogens on the aromatic ring moiety with the remainder of the molecule, or by replacing one of the alpha-hydrogens on the methyl moiety with the remainder of the molecule. Preferably, the aralkyl group is connected to the remainder of the molecule via the alkyl moiety.

A preferred aryl group is phenyl, which can be substituted or unsubstituted. Examples of substituents for substituted phenyl groups include, but are not limited to, alkyl, halogen (chlorine (-Cl), bromine (-Br), iodine (-I), or fluorine (-F)), hydroxy (-OH), or alkoxy (such as methoxy, ethoxy, n-propoxy or i-propoxy, n-butoxy, i-butoxy, sec-butoxy, or tert-butoxy). Substituted phenyl groups preferably have one or two substituents; more preferably, one substituent.

"Heteroalkyl," "heteroalkenyl," and "heteroalkynyl" refer to alkyl, alkenyl, and alkynyl groups, respectively, that contain the number of carbon atoms specified (or if no number is specified, having up to 12 carbon atoms) which contain one or more heteroatoms as part of the main, branched, or cyclic chains in the group. Heteroatoms include, but are not limited to, N, S, O, and P; N and O are preferred. Heteroalkyl, heteroalkenyl, and heteroalkynyl groups may be attached to the remainder of the molecule either at a heteroatom (if a valence is available) or at a carbon atom. Examples of heteroalkyl groups include, but are not limited to, groups such as -O-CH₃, -CH₂-O-CH₃, -CH₂-CH₂-O-CH₃, -S-CH₂-CH₂-CH₃, -CH₂-CH(CH₃)-S-CH₃, -CH₂-CH₂-NH-CH₂-CH₂-,1-ethyl-6-propylpiperidino, and morpholino. Examples of heteroalkenyl groups include, but are not limited to, groups such as -CH=CH-NH-CH(CH₃)-CH₂-. "Heteroaryl" or "HetAr" refers to an aromatic group having a single ring (including, but not limited to, examples such as pyridyl, imidazolyl, thiophene, or furyl) or two or more condensed rings (including, but not limited to, examples such as indolizinyl or benzothienyl) and having at least one hetero atom, including, but not limited to, heteroatoms such as N, O, P, or S, within the ring. Unless otherwise specified, heteroalkyl, heteroalkenyl, heteroalkynyl, and heteroaryl groups have between one and five heteroatoms and between one and twelve carbon atoms. "Substituted heteroalkyl," "substituted heteroalkenyl," "substituted heteroalkynyl," and "substituted heteroaryl" groups refer to heteroalkyl, heteroalkenyl, heteroalkynyl, and heteroaryl groups substituted with one or more substituents, including, but not limited to, groups such as alkyl, alkenyl, alkynyl, benzyl, hydrocarbon chains, halogen, alkoxy, acyloxy, amino, hydroxyl, mercapto, carboxy, benzyloxy, phenyl, benzyl, cyano, nitro, thioalkoxy, carboxaldehyde, carboalkoxy and carboxamide, or a functionality that can be suitably blocked, if necessary for purposes of the invention, with a protecting group. Examples of such substituted heteroalkyl groups include, but are not limited to, piperazine, substituted at a nitrogen or carbon by a phenyl or benzyl group, and attached to the remainder of the molecule by any available valence on a carbon or nitrogen, -NH-SO₂-phenyl, -NH-(C=O)O-alkyl, -NH-(C=O)O-alkyl-aryl, and -NH-(C=O)-alkyl. If chemically possible, the heteroatom(s) and/or the carbon atoms of the group can be substituted. The heteroatom(s) can also be in oxidized form, if chemically possible.

The term "alkoxy" as used herein refers to an alkyl, alkenyl, alkynyl, or hydrocarbon chain linked to an oxygen atom and having the number of carbon atoms specified, or if no number is specified, having up to 12 carbon atoms. Examples of alkoxy groups include, but are not limited to, groups such as methoxy, ethoxy, propyloxy (propoxy) (either n-propoxy or i-propoxy), and butoxy (either n-butoxy, i-butoxy, sec-butoxy, or tert-butoxy). The groups listed in the preceding sentence are preferred alkoxy groups; a particularly preferred alkoxy substituent is methoxy.

The terms "halo" and "halogen" as used herein refer to the Group VIIa elements (Group 17 elements in the 1990 IUPAC Periodic Table, IUPAC Nomenclature of Inorganic Chemistry, Recommendations 1990) and include Cl, Br, F and I substituents. Preferred halogen substituents are Cl and F.

When fragments, such as alkyl fragments, heteroaryl fragments, etc., are indicated as substituents, the substituent fragment can be attached to the remainder of the molecule at any point on the fragment where chemically possible (i.e., by using any available valence at a given point of the fragment, such as a valence made available by removing one or more hydrogen atoms from the fragment) unless otherwise specified. For example, in the fragment - (C=O)-C₀-C₈ alkyl-C₆-C₁₀ aryl-Co-Cs alkyl, if the leftmost C₀-C₈ alkyl group is a C₃ alkyl group, it can be attached to the sp² carbon of the carbonyl group at any of the three carbon atoms in the chain, unless otherwise specified. Likewise, the C₆-C₁₀ aryl group can be attached to the alkyl groups at any carbons in the aryl group, unless otherwise specified.

"Protecting group" refers to a chemical group that exhibits the following characteristics: 1) reacts selectively with the desired functionality in good yield to give a protected substrate that is stable to the projected reactions for which protection is desired; 2) is selectively removable from the protected substrate to yield the desired functionality; and 3) is removable in good yield by reagents compatible with the other functional group(s) present or generated in such projected reactions. Examples of suitable protecting groups can be found in Greene et al. (1991) Protective Groups in Organic Synthesis, 3rd Ed. (John Wiley & Sons, Inc., New York). Amino protecting groups include, but are not limited to, mesitylenesulfonyl (Mts), benzyloxycarbonyl (CBz or Z), t-butyloxycarbonyl (Boc), t-butyldimethylsilyl (TBS or TBDMS), 9-fluorenylmethyloxycarbonyl (Fmoc), tosyl, benzenesulfonyl, 2-pyridyl sulfonyl, or suitable photolabile protecting groups such as 6-nitroveratryloxy carbonyl (Nvoc), nitropiperonyl, pyrenylmethoxycarbonyl, nitrobenzyl, α-,α-dimethyl-dimethoxybenzyloxycarbonyl (DDZ), 5-bromo-7-nitroindolinyl, and the like. Hydroxyl protecting groups include, but are not limited to, Fmoc, TBS, photolabile protecting groups (such as nitroveratryl oxymethyl ether (Nvom)), Mom (methoxy methyl ether), and Mem (methoxy ethoxy methyl ether), NPEOC (4-nitrophenethyloxycarbonyl) and NPEOM (4-nitrophenethyloxymethyloxycarbonyl).

### Synthesis of compounds

The compounds of the invention can be readily synthesized by a variety of methods. Suitable protecting groups for reactions described herein are detailed in the text by Theodora W. Greene and Peter G. M. Wuts, Protective Groups in Organic Synthesis, 3rd edition, Hoboken, NJ: Wiley-Interscience, 1999. The syntheses below are illustrated with R₁, R₂, and R₃ as methyl; however, the methods are generally applicable when R₁, R₂, and R₃ are selected from other substituents, with suitable protection if necessary.

A method of synthesizing compounds of formula I is by adapting the following synthesis for the compound **1**: 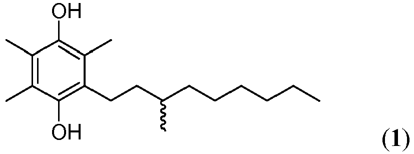 which is as follows: 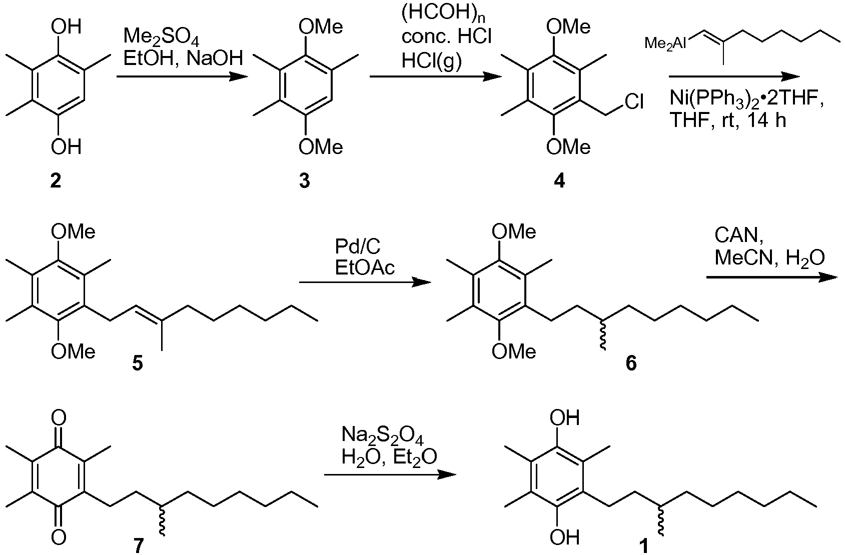 where hydroquinone **2** is dissolved in ethanol and treated with a basic solution of Me₂SO₄. Acidic workup and column chromatography yield the dimethyoxy protected hydroquinone **3**. The chloromethyl group is introduced by dissolving **3** into a solution of concentrated HCl and paraformaldehyde while adding HCl gas. Neutralization and isolation provide the product **4**. Cross-coupling according to the method outlined in Lipshutz, B.H. et.al. J.Am. Chem. Soc. 1996, 118, 5512-5513 yields the E-allylated aromatic species **5**. Compound **5** is reduced by Pd/C catalyzed hydrogenation in an appropriate solvent such as EtOH, MeOH, or EtOAc to give a racemic mixture of reduced products **6**. The protected hydroquinone is then oxidized to the quinone by treatment with CAN in acetonitrile/water mixtures to give 1,4-benzoquinone 7 directly and subsequently reduced to hydroquinone **1** by treatment of a biphasic mixutre of an etherial solvent with a basic aqueous solution of Na₂S₂O₄ (Vogel, A.I. et.al. Vogel's Textbook of Practical Organic Chemistry, 5th Edition, Prentice Hall: New York, 1996). Standard workup in the absence of oxygen yields the desired hydroquinone. Single enantiomers are available by substituting the appropriate chiral hydrogenation catalyst (Bell, S. et.al. Science 2006, 311, 642-644) in place of Pd/C.

Another method of making compounds of formula I is by adapting the following synthesis of compound **8** of the form: 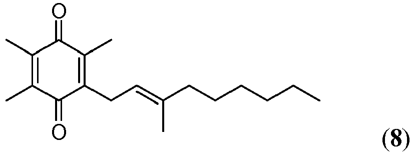 where precursor **5**, prepared as for compound **1**, is oxidized to the quinone **8** by treated with CAN in acetonitrile/water. Alternatively, quinone **8** can be prepared directly by coupling with 2-chloromethyl-3,5,6-trimethy-[1,4]-benzoquinone as described in Lipshutz, B.H. et al. Tetrahedron 1998, 54, 1241-1253.

Another method of making compounds of formula I is by adapting the following synthesis of compound **9** of the form: 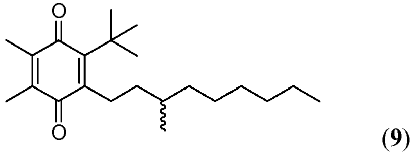 which is as follows: 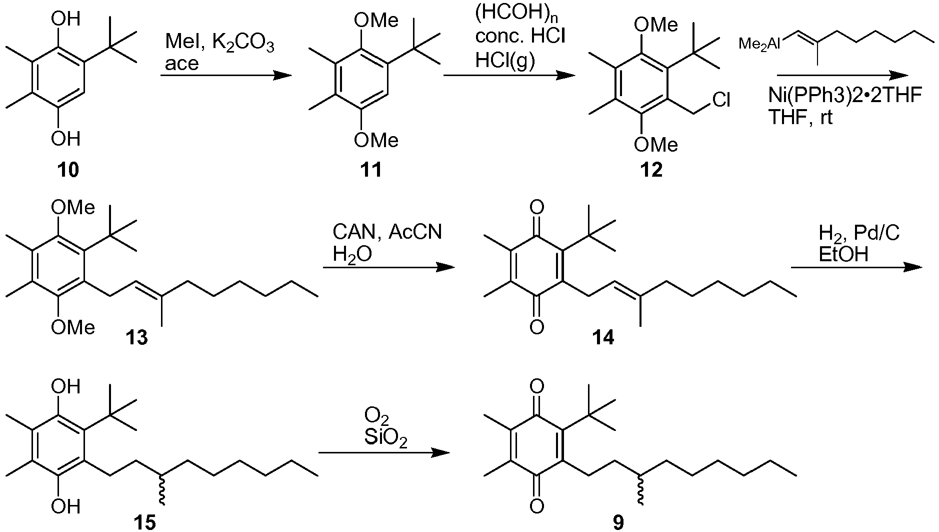 where hydroquinone **10** is methylated using methyliodide to give **11**, which is subsequently chloromethylated to provide benzylic chloride **12**. This is cross-coupled with the appropriate vinyl alane to give **13**. Compound **13** is oxidized using CAN in acetonitrile/water to provide quinone **14**, which is then exhaustively reduced by treatment with hydrogen and catalytic palladium on carbon to give hydroquinone **15**. Compound **15** is then oxidized to quinone **9** by exposure to atmospheric oxygen in the presence of silica gel. Alternatively, quinone **14** can be prepared directly by coupling with 2-chloromethyl-3-tertbutyl-5,6-dimethy-[1,4]-benzoquinone as described in Lipshutz, B.H. et al. Tetrahedron 1998, 54, 1241-1253.

Another method of making compounds of formula I is by adapting the following synthesis of compound **16** of the form: 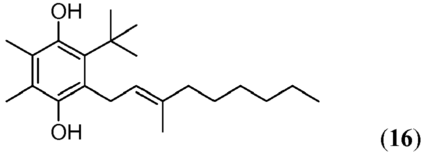 where precursor **14**, prepared as with compound **9**, is converted to the corresponding hydroquinone **16** by reduction with tin tetrachloride.

### Interconvertibility of quinone, dihydroquinone forms

The quinone and dihydroquinone forms of the compounds disclosed herein are readily interconverted with appropriate reagents. For example, the quinone form of a compound can be reduced to the dihydroquinone form with reducing agents such as sodium dithionite. The hydroquinone form can be oxidized to the quinone form with oxidizing agents such as ceric ammonium nitrate (CAN) or ferric chloride. The quinone and hydroquinone forms are also readily converted electrochemically, as is well known in the art. See, e.g., Section 33.4 of Streitweiser & Heathcock, Introduction to Organic Chemistry, New York: Macmillan, 1976.

When the compounds of the invention are drawn as the quinone or hydroquinone form, that specific form is intended. However, when the quinone form is drawn and followed by the phrase "reduced counterpart thereof' or "reduced form" or the like, the structure and the subsequent phrase are intended to embrace both the quinone and hydroquinone. Similarly, when the hydroquinone form is drawn and followed by the phrase "oxidized counterpart thereof" or "oxidized form thereof" or the like, the structure and the subsequent phrase are intended to embrace both the hydroquinone and quinone.

### Diseases amenable to treatment or suppression with compounds and methods of the invention

A variety of diseases are believed to be caused or aggravated by mitochondrial disorders and impaired energy processing, and can be treated or suppressed using the compounds of the invention. Such diseases include inherited mitochondrial diseases, such as Myoclonic Epilepsy with Ragged Red Fibers (MERRF), Mitochondrial Myopathy, Encephalopathy, Lactacidosis, Stroke (MELAS), Leber's Hereditary Optic Neuropathy (LHON, also referred to as Leber's Disease, Leber's Optic Atrophy (LOA), or Leber's Optic Neuropathy (LON)), Leigh Disease or Leigh Syndrome, Kearns-Sayre Syndrome (KSS), Friedreich's Ataxia (FA), other myopathies (including cardiomyopathy and encephalomyopathy), and renal tubular acidosis; neurodegenerative diseases, such as Parkinson's disease, Alzheimer's disease, amyotrophic lateral sclerosis (ALS, also known as Lou Gehrig's disease), motor neuron diseases; other neurological diseases such as epilepsy; genetic diseases such as Huntington's Disease (which is also a neurological disease); mood disorders such as schizophrenia and bipolar disorder; and certain age-associated diseases, particularly diseases for which CoQ10 has been proposed for treatment, such as macular degeneration, diabetes, and cancer.

### In vitro assessment of efficacy of compounds

The compounds of the invention can be tested *in vitro* for efficacy. One such assay is ability of a compound to rescue FRDA fibroblasts stressed by addition of L-buthionine-(S,R)-sulfoximine (BSO), as described in Jauslin et al., Hum. Mol. Genet. 11(24):3055 (2002), Jauslin et al., FASEB J. 17:1972-4 (2003), and International Patent Application WO 2004/003565. Human dermal fibroblasts from Friedreich's Ataxia patients have been shown to be hypersensitive to inhibition of the *de novo* synthesis of glutathione (GSH) with L-buthionine-(S,R)-sulfoximine (BSO), a specific inhibitor of GSH synthetase (Jauslin et al., Hum. Mol. Genet. 11(24):3055 (2002)). This specific BSO-mediated cell death can be prevented by administration of antioxidants or molecules involved in the antioxidant pathway, such as α-tocopherol, short chain quinones, selenium, or small molecule glutathione peroxidase mimetics. However, antioxidants differ in their potency, i.e. the concentration at which they are able to rescue BSO-stressed FRDA fibroblasts. With this assay, EC₅₀ concentrations of the compounds of the invention can be determined and compared to known reference antioxidants.

### Clinical assessment of mitochondrial dysfunction and efficacy of therapy

Several readily measurable clinical markers are used to assess the metabolic state of patients with mitochondrial disorders. These markers can also be used as indicators of the efficacy of a given therapy, as the level of a marker is moved from the pathological value to the healthy value. These clinical markers include, but are not limited to, one or more of the previously discussed energy biomarkers, such as lactic acid (lactate) levels, either in whole blood, plasma, cerebrospinal fluid, or cerebral ventricular fluid; pyruvic acid (pyruvate) levels, either in whole blood, plasma, cerebrospinal fluid, or cerebral ventricular fluid; lactate/pyruvate ratios, either in whole blood, plasma, cerebrospinal fluid, or cerebral ventricular fluid; phosphocreatine levels, NADH (NADH +H⁺) or NADPH (NADPH+H⁺) levels; NAD or NADP levels; ATP levels; anaerobic threshold; reduced coenzyme Q (CoQ^{red}) levels; oxidized coenzyme Q (CoQ^{ox}) levels; total coenzyme Q (CoQ^{tot}) levels; oxidized cytochrome C levels; reduced cytochrome C levels; oxidized cytochrome C/reduced cytochrome C ratio; acetoacetate levels, β-hydroxy butyrate levels, acetoacetate/β-hydroxy butyrate ratio, 8-hydroxy-2'-deoxyguanosine (8-OHdG) levels; levels of reactive oxygen species; and levels of oxygen consumption (VO2), levels of carbon dioxide output (VCO2), and respiratory quotient (VCO2/VO2). Several of these clinical markers are measured routinely in exercise physiology laboratories, and provide convenient assessments of the metabolic state of a subject. In one embodiment of the invention, the level of one or more energy biomarkers in a patient suffering from a mitochondrial disease, such as Friedreich's ataxia, Leber's hereditary optic neuropathy, MELAS, or KSS, is improved to within two standard deviations of the average level in a healthy subject. In another embodiment of the invention, the level of one or more of these energy biomarkers in a patient suffering from a mitochondrial disease, such as Friedreich's ataxia, Leber's hereditary optic neuropathy, MELAS, or KSS is improved to within one standard deviation of the average level in a healthy subject. Exercise intolerance can also be used as an indicator of the efficacy of a given therapy, where an improvement in exercise tolerance (i.e., a decrease in exercise intolerance) indicates efficacy of a given therapy.

Several metabolic biomarkers have already been used to evaluate efficacy of CoQ10, and these metabolic biomarkers can be monitored as energy biomarkers for use in the methods of the current invention. Pyruvate, a product of the anaerobic metabolism of glucose, is removed by reduction to lactic acid in an anaerobic setting or by oxidative metabolism, which is dependent on a functional mitochondrial respiratory chain. Dysfunction of the respiratory chain may lead to inadequate removal of lactate and pyruvate from the circulation and elevated lactate/pyruvate ratios are observed in mitochondrial cytopathies (see Scriver CR, The metabolic and molecular bases of inherited disease, 7th ed., New York: McGraw-Hill, Health Professions Division, 1995; and Munnich et al., J. Inherit. Metab. Dis. 15(4):448-55 (1992)). Blood lactate/pyruvate ratio (Chariot et al., Arch. Pathol. Lab. Med. 118(7):695-7 (1994)) is, therefore, widely used as a noninvasive test for detection of mitochondrial cytopathies (see again Scriver CR, The metabolic and molecular bases of inherited disease, 7th ed., New York: McGraw-Hill, Health Professions Division, 1995; and Munnich et al., J. Inherit. Metab. Dis. 15(4):448-55 (1992)) and toxic mitochondrial myopathies (Chariot et al., Arthritis Rheum. 37(4):583-6 (1994)). Changes in the redox state of liver mitochondria can be investigated by measuring the arterial ketone body ratio (acetoacetate/3-hydroxybutyrate: AKBR) (Ueda et al., J. Cardiol. 29(2):95-102 (1997)). Urinary excretion of 8-hydroxy-2'-deoxyguanosine (8-OHdG) often has been used as a biomarker to assess the extent of repair of ROS-induced DNA damage in both clinical and occupational settings (Erhola et al., FEBS Lett. 409(2):287-91 (1997); Honda et al., Leuk. Res. 24(6):461-8 (2000); Pilger et al., Free Radic. Res. 35(3):273-80 (2001); Kim et al. Environ Health Perspect 112(6):666-71 (2004)).

Magnetic resonance spectroscopy (MRS) has been useful in the diagnoses of mitochondrial cytopathy by demonstrating elevations in cerebrospinal fluid (CSF) and cortical white matter lactate using proton MRS (1H-MRS) (Kaufmann et al., Neurology 62(8):1297-302 (2004)). Phosphorous MRS (31P-MRS) has been used to demonstrate low levels of cortical phosphocreatine (PCr) (Matthews et al., Ann. Neurol. 29(4):435-8 (1991)), and a delay in PCr recovery kinetics following exercise in skeletal muscle (Matthews et al., Ann. Neurol. 29(4):435-8 (1991); Barbiroli et al., J. Neurol. 242(7):472-7 (1995); Fabrizi et al., J. Neurol. Sci. 137(1):20-7 (1996)). A low skeletal muscle PCr has also been confirmed in patients with mitochondrial cytopathy by direct biochemical measurements.

Exercise testing is particularly helpful as an evaluation and screening tool in mitochondrial myopathies. One of the hallmark characteristics of mitochondrial myopathies is a reduction in maximal whole body oxygen consumption (VO2max) (Taivassalo et al.,. Brain 126(Pt 2):413-23 (2003)). Given that VO2max is determined by cardiac output (Qc) and peripheral oxygen extraction (arterial-venous total oxygen content) difference, some mitochondrial cytopathies affect cardiac function where delivery can be altered; however, most mitochondrial myopathies show a characteristic deficit in peripheral oxygen extraction (A-V O2 difference) and an enhanced oxygen delivery (hyperkinetic circulation) (Taivassalo et al.,. Brain 126(Pt 2):413-23 (2003)). This can be demonstrated by a lack of exercise induced deoxygenation of venous blood with direct AV balance measurements (Taivassalo et al., Ann. Neurol. 51(1):38-44 (2002)) and non-invasively by near infrared spectroscopy (Lynch et al., Muscle Nerve 25(5):664-73 (2002); van Beekvelt et al., Ann. Neurol. 46(4):667-70 (1999)).

Several of these energy biomarkers are discussed in more detail as follows. It should be emphasized that, while certain energy biomarkers are discussed and enumerated herein, the invention is not limited to modulation, normalization or enhancement of only these enumerated energy biomarkers.

*Lactic acid (lactate) levels:* Mitochondrial dysfunction typically results in abnormal levels of lactic acid, as pyruvate levels increase and pyruvate is converted to lactate to maintain capacity for glycolysis. Mitochondrial dysfunction can also result in abnormal levels of NADH +H⁺, NADPH+H⁺, NAD, or NADP, as the reduced nicotinamide adenine dinucleotides are not efficiently processed by the respiratory chain. Lactate levels can be measured by taking samples of appropriate bodily fluids such as whole blood, plasma, or cerebrospinal fluid. Using magnetic resonance, lactate levels can be measured in virtually any volume of the body desired, such as the brain.

Measurement of cerebral lactic acidosis using magnetic resonance in MELAS patients is described in Kaufmann et al., Neurology 62(8):1297 (2004). Values of the levels of lactic acid in the lateral ventricles of the brain are presented for two mutations resulting in MELAS, A3243G and A8344G. Whole blood, plasma, and cerebrospinal fluid lactate levels can be measured by commercially available equipment such as the YSI 2300 STAT Plus Glucose & Lactate Analyzer (YSI Life Sciences, Ohio).

*NAD, NADP, NADH and NADPH levels*: Measurement of NAD, NADP, NADH (NADH +H⁺) or NADPH (NADPH+H⁺) can be measured by a variety of fluorescent, enzymatic, or electrochemical techniques, e.g., the electrochemical assay described in US 2005/0067303.

*Oxygen consumption* (*vO₂ or VO2*), *carbon dioxide output* (*vCO₂ or VCO2*), *and respiratory quotient* (*VCO2*/*VO2*): vO₂ is usually measured either while resting (resting vO₂) or at maximal exercise intensity (vO₂ max). Optimally, both values will be measured. However, for severely disabled patients, measurement of vO₂ max may be impractical. Measurement of both forms of vO₂ is readily accomplished using standard equipment from a variety of vendors, e.g. Korr Medical Technologies, Inc. (Salt Lake City, Utah). VCO2 can also be readily measured, and the ratio of VCO2 to VO2 under the same conditions (VCO2/VO2, either resting or at maximal exercise intensity) provides the respiratory quotient (RQ).

*Oxidized Cytochrome C, reduced Cytochrome C, and ratio of oxidized Cytochrome C to reduced Cytochrome C*: Cytochrome C parameters, such as oxidized cytochrome C levels (Cyt Cₒₓ), reduced cytochrome C levels (Cyt C_{red}), and the ratio of oxidized cytochrome C/reduced cytochrome C ratio (Cyt Cₒₓ)/(Cyt C_{red}), can be measured by in vivo near infrared spectroscopy. See, e.g., Rolfe, P., "In vivo near-infrared spectroscopy," Annu. Rev. Biomed. Eng. 2:715-54 (2000) and Strangman et al., "Non-invasive neuroimaging using near-infrared light" Biol. Psychiatry 52:679-93 (2002).

*Exercise tolerance*/*Exercise intolerance*: Exercise intolerance is defined as "the reduced ability to perform activities that involve dynamic movement of large skeletal muscles because of symptoms of dyspnea or fatigue" (Piña et al., Circulation 107:1210 (2003)). Exercise intolerance is often accompanied by myoglobinuria, due to breakdown of muscle tissue and subsequent excretion of muscle myoglobin in the urine. Various measures of exercise intolerance can be used, such as time spent walking or running on a treadmill before exhaustion, time spent on an exercise bicycle (stationary bicycle) before exhaustion, and the like. Treatment with the compounds of the invention can result in about a 10% or greater improvement in exercise tolerance (for example, about a 10% or greater increase in time to exhaustion, e.g. from 10 minutes to 11 minutes), about a 20% or greater improvement in exercise tolerance, about a 30% or greater improvement in exercise tolerance, about a 40% or greater improvement in exercise tolerance, about a 50% or greater improvement in exercise tolerance, about a 75% or greater improvement in exercise tolerance, or about a 100% or greater improvement in exercise tolerance. While exercise tolerance is not, strictly speaking, an energy biomarker, for the purposes of the invention, modulation, normalization, or enhancement of energy biomarkers includes modulation, normalization, or enhancement of exercise tolerance.

Similarly, tests for normal and abnormal values of pyruvic acid (pyruvate) levels, lactate/pyruvate ratio, ATP levels, anaerobic threshold, reduced coenzyme Q (CoQ^{red}) levels, oxidized coenzyme Q (CoQ^{ox}) levels, total coenzyme Q (CoQ^{tot}) levels, oxidized cytochrome C levels, reduced cytochrome C levels, oxidized cytochrome C/reduced cytochrome C ratio, acetoacetate levels, β-hydroxy butyrate levels, acetoacetate/β-hydroxy butyrate ratio, 8-hydroxy-2'-deoxyguanosine (8-OHdG) levels, and levels of reactive oxygen species are known in the art and can be used to evaluate efficacy of the compounds and methods of the invention. (For the purposes of the invention, modulation, normalization, or enhancement of energy biomarkers includes modulation, normalization, or enhancement of anaerobic threshold.)

Table 1, following, illustrates the effect that various dysfunctions can have on biochemistry and energy biomarkers. It also indicates the physical effect (such as a disease symptom or other effect of the dysfunction) typically associated with a given dysfunction. It should be noted that any of the energy biomarkers listed in the table, in addition to energy biomarkers enumerated elsewhere, can also be modulated, enhanced, or normalized by the compounds of the invention. RQ = respiratory quotient; BMR = basal metabolic rate; HR (CO) = heart rate (cardiac output); T = body temperature (preferably measured as core temperature); AT = anaerobic threshold; pH = blood pH (venous and/or arterial).

**Table 1**

| Site of Dysfunction | Biochemical Event | Measurable Energy Biomarker | Physical Effect |
|---|---|---|---|
| Respiratory Chain | ↑ NADH | Δ lactate, | Metabolic dyscrasia & fatigue |
| | | Δ lactate: pyruvate ratio; and | |
| | | Δ acetoacetate: β-hydroxy butyrate ratio | |
| Respiratory Chain | ↓ H⁺ gradient | Δ ATP | Organ dependent dysfunction |
| Respiratory Chain | ↓ Electron flux | Δ VO₂, RQ, BMR, ΔT, AT, pH | Metabolic dyscrasia & fatigue |
| Mitochondria & cytosol | ↓ ATP, ↓ VO₂ | Δ Work, ΔHR (CO) | Exercise intolerance |
| Mitochondria & cytosol | ↓ ATP | Δ PCr | Exercise intolerance |
| Respiratory Chain | ↓ Cyt C_{Ox/Red} | Δ λ ∼700 - 900 nM (Near Infrared Spectroscopy) | Exercise intolerance |
| Intermediary metabolism | ↓ *Catabolism* | Δ C¹⁴-Labeled substrates | Metabolic dyscrasia & fatigue |
| Respiratory Chain | ↓ Electron flux | Δ Mixed Venous VO₂ | Metabolic dyscrasia & fatigue |
| Mitochondria & cytosol | ↑ Oxidative stress | Δ Tocopherol & Tocotrienols, CoQ10, docosahexanoic acid | Uncertain |
| Mitochondria & cytosol | ↑ Oxidative stress | Δ Glutathione_{red} | Uncertain |
| Mitochondria & cytosol | Nucleic acid oxidation | 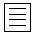 8-hydroxy 2-deoxy guanosine | Uncertain |
| Mitochondria & cytosol | Lipid oxidation | ΔIsoprostane(s), eicasanoids | Uncertain |
| Cell membranes | Lipid oxidation | ΔEthane (breath) | Uncertain |
| Cell membranes | Lipid oxidation | ΔMalondialdehyde | Uncertain |

Treatment of a subject afflicted by a mitochondrial disease in accordance with the compounds of the invention may result in the inducement of a reduction or alleviation of symptoms in the subject, e.g., to halt the further progression of the disorder.

Partial or complete suppression of the mitochondrial disease can result in a lessening of the severity of one or more of the symptoms that the subject would otherwise experience. For example, partial suppression of MELAS could result in reduction in the number of stroke-like or seizure episodes suffered.

Any one, or any combination of, the energy biomarkers described herein provide conveniently measurable benchmarks by which to gauge the effectiveness of treatment or suppressive therapy. Additionally, other energy biomarkers are known to those skilled in the art and can be monitored to evaluate the efficacy of treatment or suppressive therapy.

### Use of compounds for modulation of energy biomarkers

In addition to monitoring energy biomarkers to assess the status of treatment or suppression of mitochondrial diseases, the compounds of the invention can be used in subjects or patients to modulate one or more energy biomarkers. Modulation of energy biomarkers can be done to normalize energy biomarkers in a subject, or to enhance energy biomarkers in a subject.

Normalization of one or more energy biomarkers is defined as either restoring the level of one or more such energy biomarkers to normal or near-normal levels in a subject whose levels of one or more energy biomarkers show pathological differences from normal levels (i.e., levels in a healthy subject), or to change the levels of one or more energy biomarkers to alleviate pathological symptoms in a subject. Depending on the nature of the energy biomarker, such levels may show measured values either above or below a normal value. For example, a pathological lactate level is typically higher than the lactate level in a normal (i.e., healthy) person, and a decrease in the level may be desirable. A pathological ATP level is typically lower than the ATP level in a normal (i.e., healthy) person, and an increase in the level of ATP may be desirable. Accordingly, normalization of energy biomarkers can involve restoring the level of energy biomarkers to within about at least two standard deviations of normal in a subject, more preferably to within about at least one standard deviation of normal in a subject, to within about at least one-half standard deviation of normal, or to within about at least one-quarter standard deviation of normal.

When an increase in an energy biomarker level is desired to normalize the one or more such energy biomarker, the level of the energy biomarker can be increased to within about at least two standard deviations of normal in a subject, more preferably increased to within about at least one standard deviation of normal in a subject, increased to within about at least one-half standard deviation of normal, or increased to within about at least one-quarter standard deviation of normal, by administration of one or more compounds according to the invention. Alternatively, the level of one or more of the energy biomarkers can be increased by about at least 10% above the subject's level of the respective one or more energy biomarkers before administration, by about at least 20% above the subject's level of the respective one or more energy biomarkers before administration, by about at least 30% above the subject's level of the respective one or more energy biomarkers before administration, by about at least 40% above the subject's level of the respective one or more energy biomarkers before administration, by about at least 50% above the subject's level of the respective one or more energy biomarkers before administration, by about at least 75% above the subject's level of the respective one or more energy biomarkers before administration, or by about at least 100% above the subject's level of the respective one or more energy biomarkers before administration.

When a decrease in a level of one or more energy biomarkers is desired to normalize the one or more energy biomarkers, the level of the one or more energy biomarkers can be decreased to a level within about at least two standard deviations of normal in a subject, more preferably decreased to within about at least one standard deviation of normal in a subject, decreased to within about at least one-half standard deviation of normal, or decreased to within about at least one-quarter standard deviation of normal, by administration of one or more compounds according to the invention. Alternatively, the level of the one or more energy biomarkers can be decreased by about at least 10% below the subject's level of the respective one or more energy biomarkers before administration, by about at least 20% below the subject's level of the respective one or more energy biomarkers before administration, by about at least 30% below the subject's level of the respective one or more energy biomarkers before administration, by about at least 40% below the subject's level of the respective one or more energy biomarkers before administration, by about at least 50% below the subject's level of the respective one or more energy biomarkers before administration, by about at least 75% below the subject's level of the respective one or more energy biomarkers before administration, or by about at least 90% below the subject's level of the respective one or more energy biomarkers before administration.

Enhancement of the level of one or more energy biomarkers is defined as changing the extant levels of one or more energy biomarkers in a subject to a level which provides beneficial or desired effects for the subject. For example, a person undergoing strenuous effort or prolonged vigorous physical activity, such as mountain climbing, could benefit from increased ATP levels or decreased lactate levels. As described above, normalization of energy biomarkers may not achieve the optimum state for a subject with a mitochondrial disease, and such subjects can also benefit from enhancement of energy biomarkers. Examples of subjects who could benefit from enhanced levels of one or more energy biomarkers include, but are not limited to, subjects undergoing strenuous or prolonged physical activity, subjects with chronic energy problems, or subjects with chronic respiratory problems. Such subjects include, but are not limited to, pregnant females, particularly pregnant females in labor; neonates, particularly premature neonates; subjects exposed to extreme environments, such as hot environments (temperatures routinely exceeding about 85-86 degrees Fahrenheit or about 30 degrees Celsius for about 4 hours daily or more), cold environments (temperatures routinely below about 32 degrees Fahrenheit or about 0 degrees Celsius for about 4 hours daily or more), or environments with lower-than-average oxygen content, higher-than-average carbon dioxide content, or higher-than-average levels of air pollution (airline travelers, flight attendants, subjects at elevated altitudes, subjects living in cities with lower-than-average air quality, subjects working in enclosed environments where air quality is degraded); subjects with lung diseases or lower-than-average lung capacity, such as tubercular patients, lung cancer patients, emphysema patients, and cystic fibrosis patients; subjects recovering from surgery or illness; elderly subjects, including elderly subjects experiencing decreased energy; subjects suffering from chronic fatigue, including chronic fatigue syndrome; subjects undergoing acute trauma; subjects in shock; subjects requiring acute oxygen administration; subjects requiring chronic oxygen administration; or other subjects with acute, chronic, or ongoing energy demands who can benefit from enhancement of energy biomarkers.

Accordingly, when an increase in a level of one or more energy biomarkers is beneficial to a subject, enhancement of the one or more energy biomarkers can involve increasing the level of the respective energy biomarker or energy biomarkers to about at least one-quarter standard deviation above normal, about at least one-half standard deviation above normal, about at least one standard deviation above normal, or about at least two standard deviations above normal. Alternatively, the level of the one or more energy biomarkers can be increased by about at least 10% above the subject's level of the respective one or more energy biomarkers before enhancement, by about at least 20% above the subject's level of the respective one or more energy biomarkers before enhancement, by about at least 30% above the subject's level of the respective one or more energy biomarkers before enhancement, by about at least 40% above the subject's level of the respective one or more energy biomarkers before enhancement, by about at least 50% above the subject's level of the respective one or more energy biomarkers before enhancement, by about at least 75% above the subject's level of the respective one or more energy biomarkers before enhancement, or by about at least 100% above the subject's level of the respective one or more energy biomarkers before enhancement.

When a decrease in a level of one or more energy biomarkers is desired to enhance one or more energy biomarkers, the level of the one or more energy biomarkers can be decreased by an amount of about at least one-quarter standard deviation of normal in a subject, decreased by about at least one-half standard deviation of normal in a subject, decreased by about at least one standard deviation of normal in a subject, or decreased by about at least two standard deviations of normal in a subject. Alternatively, the level of the one or more energy biomarkers can be decreased by about at least 10% below the subject's level of the respective one or more energy biomarkers before enhancement, by about at least 20% below the subject's level of the respective one or more energy biomarkers before enhancement, by about at least 30% below the subject's level of the respective one or more energy biomarkers before enhancement, by about at least 40% below the subject's level of the respective one or more energy biomarkers before enhancement, by about at least 50% below the subject's level of the respective one or more energy biomarkers before enhancement, by about at least 75% below the subject's level of the respective one or more energy biomarkers before enhancement, or by about at least 90% below the subject's level of the respective one or more energy biomarkers before enhancement.

### Use of compounds in research applications, experimental systems, and assays

The compounds of the invention can also be used in research applications, such as in *in vitro, in vivo,* or *ex vivo* experiments in order to modulate one or more energy biomarkers in an experimental system. Such experimental systems can be cell samples, tissue samples, cell components or mixtures of cell components, partial organs, whole organs, or organisms. Such research applications can include, but are not limited to, use as assay reagents, elucidation of biochemical pathways, or evaluation of the effects of other agents on the metabolic state of the experimental system in the presence/absence of one or more compounds of the invention.

Additionally, the compounds of the invention can be used in biochemical tests or assays. Such tests can include incubation of one or more compounds of the invention with a tissue or cell sample from a subject to evaluate a subject's potential response (or the response of a specific subset of subjects) to administration of said one or more compounds, or to determine which compound of the invention produces the optimum effect in a specific subject or subset of subjects. One such test or assay would involve 1) obtaining a cell sample or tissue sample from a subject or set of subjects in which modulation of one or more energy biomarkers can be assayed; 2) administering one or more compounds of the invention to the cell sample(s) or tissue sample(s); and 3) determining the amount of modulation of the one or more energy biomarkers after administration of the one or more compounds, compared to the status of the energy biomarker prior to administration of the one or more compounds. Another such test or assay would involve 1) obtaining a cell sample or tissue sample from a subject or set of subjects in which modulation of one or more energy biomarkers can be assayed; 2) administering at least two compounds of the invention to the cell sample(s) or tissue sample(s); 3) determining the amount of modulation of the one or more energy biomarkers after administration of the at least two compounds, compared to the status of the energy biomarker prior to administration of the at least two compounds, and 4) selecting a compound for use in treatment, suppression, or modulation based on the amount of modulation determined in step 3).

### Pharmaceutical formulations

The compounds described herein can be formulated as pharmaceutical compositions by formulation with additives such as pharmaceutically acceptable excipients, pharmaceutically acceptable carriers, and pharmaceutically acceptable vehicles. Suitable pharmaceutically acceptable excipients, carriers and vehicles include processing agents and drug delivery modifiers and enhancers, such as, for example, calcium phosphate, magnesium stearate, talc, monosaccharides, disaccharides, starch, gelatin, cellulose, methyl cellulose, sodium carboxymethyl cellulose, dextrose, hydroxypropyl-β-cyclodextrin, polyvinylpyrrolidinone, low melting waxes, ion exchange resins, and the like, as well as combinations of any two or more thereof. Other suitable pharmaceutically acceptable excipients are described in "Remington's Pharmaceutical Sciences," Mack Pub. Co., New Jersey (1991), and "Remington: The Science and Practice of Pharmacy," Lippincott Williams & Wilkins, Philadelphia, 20th edition (2003) and 21st edition (2005).

A pharmaceutical composition can comprise a unit dose formulation, where the unit dose is a dose sufficient to have a therapeutic or suppressive effect or an amount effective to modulate, normalize, or enhance an energy biomarker. The unit dose may be sufficient as a single dose to have a therapeutic or suppressive effect or an amount effective to modulate, normalize, or enhance an energy biomarker. Alternatively, the unit dose may be a dose administered periodically in a course of treatment or suppression of a disorder, or to modulate, normalize, or enhance an energy biomarker.

Pharmaceutical compositions containing the compounds of the invention may be in any form suitable for the intended method of administration, including, for example, a solution, a suspension, or an emulsion. Liquid carriers are typically used in preparing solutions, suspensions, and emulsions. Liquid carriers contemplated for use in the practice of the present invention include, for example, water, saline, pharmaceutically acceptable organic solvent(s), pharmaceutically acceptable oils or fats, and the like, as well as mixtures of two or more thereof. The liquid carrier may contain other suitable pharmaceutically acceptable additives such as solubilizers, emulsifiers, nutrients, buffers, preservatives, suspending agents, thickening agents, viscosity regulators, stabilizers, and the like. Suitable organic solvents include, for example, monohydric alcohols, such as ethanol, and polyhydric alcohols, such as glycols. Suitable oils include, for example, soybean oil, coconut oil, olive oil, safflower oil, cottonseed oil, and the like. For parenteral administration, the carrier can also be an oily ester such as ethyl oleate, isopropyl myristate, and the like. Compositions of the present invention may also be in the form of microparticles, microcapsules, liposomal encapsulates, and the like, as well as combinations of any two or more thereof.

Time-release or controlled release delivery systems may be used, such as a diffusion controlled matrix system or an erodible system, as described for example in: Lee, "Diffusion-Controlled Matrix Systems", pp. 155-198 and Ron and Langer, "Erodible Systems", pp. 199-224, in "Treatise on Controlled Drug Delivery", A. Kydonieus Ed., Marcel Dekker, Inc., New York 1992. The matrix may be, for example, a biodegradable material that can degrade spontaneously *in situ* and *in vivo* for, example, by hydrolysis or enzymatic cleavage, e.g., by proteases. The delivery system may be, for example, a naturally occurring or synthetic polymer or copolymer, for example in the form of a hydrogel. Exemplary polymers with cleavable linkages include polyesters, polyorthoesters, polyanhydrides, polysaccharides, poly(phosphoesters), polyamides, polyurethanes, poly(imidocarbonates) and poly(phosphazenes).

The compounds of the invention may be administered enterally, orally, parenterally, sublingually, by inhalation (e.g. as mists or sprays), rectally, or topically in dosage unit formulations containing conventional nontoxic pharmaceutically acceptable carriers, adjuvants, and vehicles as desired. For example, suitable modes of administration include oral, subcutaneous, transdermal, transmucosal, iontophoretic, intravenous, intraarterial, intramuscular, intraperitoneal, intranasal (e.g. via nasal mucosa), subdural, rectal, gastrointestinal, and the like, and directly to a specific or affected organ or tissue. For delivery to the central nervous system, spinal and epidural administration, or administration to cerebral ventricles, can be used. Topical administration may also involve the use of transdermal administration such as transdermal patches or iontophoresis devices. The term parenteral as used herein includes subcutaneous injections, intravenous, intramuscular, intrasternal injection, or infusion techniques. The compounds are mixed with pharmaceutically acceptable carriers, adjuvants, and vehicles appropriate for the desired route of administration. Oral administration is a preferred route of administration, and formulations suitable for oral administration are preferred formulations. The compounds described for use herein can be administered in solid form, in liquid form, in aerosol form, or in the form of tablets, pills, powder mixtures, capsules, granules, injectables, creams, solutions, suppositories, enemas, colonic irrigations, emulsions, dispersions, food premixes, and in other suitable forms. The compounds can also be administered in liposome formulations. Additional methods of administration are known in the art.

Injectable preparations, for example, sterile injectable aqueous or oleaginous suspensions, may be formulated according to the known art using suitable dispersing or wetting agents and suspending agents. The sterile injectable preparation may also be a sterile injectable solution or suspension in a nontoxic parenterally acceptable diluent or solvent, for example, as a solution in propylene glycol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

Suppositories for rectal administration of the drug can be prepared by mixing the drug with a suitable nonirritating excipient such as cocoa butter and polyethylene glycols that are solid at room temperature but liquid at the rectal temperature and will therefore melt in the rectum and release the drug.

Solid dosage forms for oral administration may include capsules, tablets, pills, powders, and granules. In such solid dosage forms, the active compound may be admixed with at least one inert diluent such as sucrose, lactose, or starch. Such dosage forms may also comprise additional substances other than inert diluents, e.g., lubricating agents such as magnesium stearate. In the case of capsules, tablets, and pills, the dosage forms may also comprise buffering agents. Tablets and pills can additionally be prepared with enteric coatings.

Liquid dosage forms for oral administration may include pharmaceutically acceptable emulsions, solutions, suspensions, syrups, and elixirs containing inert diluents commonly used in the art, such as water. Such compositions may also comprise adjuvants, such as wetting agents, emulsifying and suspending agents, cyclodextrins, and sweetening, flavoring, and perfuming agents.

The compounds of the present invention can also be administered in the form of liposomes. As is known in the art, liposomes are generally derived from phospholipids or other lipid substances. Liposomes are formed by mono- or multilamellar hydrated liquid crystals that are dispersed in an aqueous medium. Any non-toxic, physiologically acceptable and metabolizable lipid capable of forming liposomes can be used. The present compositions in liposome form can contain, in addition to a compound of the present invention, stabilizers, preservatives, excipients, and the like. The preferred lipids are the phospholipids and phosphatidyl cholines (lecithins), both natural and synthetic. Methods to form liposomes are known in the art. See, for example, Prescott, Ed., Methods in Cell Biology, Volume XIV, Academic Press, New York, N.W., p. 33 et seq (1976).

Articles of manufacture and kits containing materials useful for treating or suppressing mitochondrial diseases may be prepared. The article of manufacture comprises a container with a label. Suitable containers include, for example, bottles, vials, and test tubes. The containers may be formed from a variety of materials such as glass or plastic. The container holds a composition having an active agent which is effective for treating or suppressing mitochondrial diseases. The active agent in the composition is one or more of the compounds of the invention. The label on the container indicates that the composition is used for treating or suppressing mitochondrial diseases, and may also indicate directions for either *in vivo* or *in vitro* use, such as those described above.

Kits comprising any one or more of the compounds of the invention may be prepared. In some embodiments, the kit comprises the container described above. In other embodiments, the kit comprises the container described above and a second container comprising a buffer. It may further include other materials desirable from a commercial and user standpoint, including other buffers, diluents, filters, needles, syringes, and package inserts with instructions for performing any methods described herein.

In other aspects, the kits may be used for any of the methods described herein, including, for example, to treat an individual with a mitochondrial disorder, or to suppress a mitochondrial disorder in an individual.

The amount of active ingredient that may be combined with the carrier materials to produce a single dosage form will vary depending upon the host to which the active ingredient is administered and the particular mode of administration. It will be understood, however, that the specific dose level for any particular patient will depend upon a variety of factors including the activity of the specific compound employed, the age, body weight, body area, body mass index (BMI), general health, sex, diet, time of administration, route of administration, rate of excretion, drug combination, and the type, progression, and severity of the particular disease undergoing therapy. The pharmaceutical unit dosage chosen is usually fabricated and administered to provide a defined final concentration of drug in the blood, tissues, organs, or other targeted region of the body. The therapeutically effective amount or effective amount for a given situation can be readily determined by routine experimentation and is within the skill and judgment of the ordinary clinician.

Examples of dosages which can be used are an effective amount within the dosage range of about 0.1 µg/kg to about 300 mg/kg, or within about 1.0 µg/kg to about 40 mg/kg body weight, or within about 1.0 µg/kg to about 20 mg/kg body weight, or within about 1.0 µg/kg to about 10 mg/kg body weight, or within about 10.0 µg/kg to about 10 mg/kg body weight, or within about 100 µg/kg to about 10 mg/kg body weight, or within about 1.0 mg/kg to about 10 mg/kg body weight, or within about 10 mg/kg to about 100 mg/kg body weight, or within about 50 mg/kg to about 150 mg/kg body weight, or within about 100 mg/kg to about 200 mg/kg body weight, or within about 150 mg/kg to about 250 mg/kg body weight, or within about 200 mg/kg to about 300 mg/kg body weight, or within about 250 mg/kg to about 300 mg/kg body weight. Other dosages which can be used are about 0.01 mg/kg body weight, about 0.1 mg/kg body weight, about 1 mg/kg body weight, about 10 mg/kg body weight, about 20 mg/kg body weight, about 30 mg/kg body weight, about 40 mg/kg body weight, about 50 mg/kg body weight, about 75 mg/kg body weight, about 100 mg/kg body weight, about 125 mg/kg body weight, about 150 mg/kg body weight, about 175 mg/kg body weight, about 200 mg/kg body weight, about 225 mg/kg body weight, about 250 mg/kg body weight, about 275 mg/kg body weight, or about 300 mg/kg body weight. Compounds of the present invention may be administered in a single daily dose, or the total daily dosage may be administered in divided dosage of two, three or four times daily.

While the compounds of the invention can be administered as the sole active pharmaceutical agent, they can also be used in combination with one or more other agents used in the treatment or suppression of disorders. Representative agents useful in combination with the compounds of the invention for the treatment or suppression of mitochondrial diseases include, but are not limited to, Coenzyme Q, vitamin E, idebenone, MitoQ, vitamins, and antioxidant compounds.

When additional active agents are used in combination with the compounds of the present invention, the additional active agents may generally be employed in therapeutic amounts as indicated in the Physicians' Desk Reference (PDR) 53rd Edition (1999), or such therapeutically useful amounts as would be known to one of ordinary skill in the art.

The compounds of the invention and the other therapeutically active agents can be administered at the recommended maximum clinical dosage or at lower doses. Dosage levels of the active compounds in the compositions of the invention may be varied so as to obtain a desired therapeutic response depending on the route of administration, severity of the disease and the response of the patient. When administered in combination with other therapeutic agents, the therapeutic agents can be formulated as separate compositions that are given at the same time or different times, or the therapeutic agents can be given as a single composition.

The invention will be further understood by the following nonlimiting examples.

### EXAMPLES

### Example 1

### Synthesis of compounds

### 2,3-Dimethyl-5,6-bis-(3-methyl-butyl)-[1,4]benzoquinone (not of the invention)

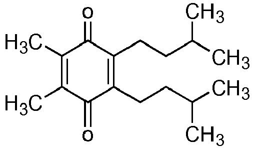

A solution of FeCl₃·6H₂O (81.0g, 300mmol) in water (100mL) was added to a solution of 2,3-dimethyl-benzene-1,4-diol (13.8g, 100 mmol) in MTBE (150 ml) at ambient temperature. Aqueous sodium hydroxide solution (2.5M, 60mL, 150mmol) was added to the vigorously stirring mixture and the reaction heated to 50°C for 5 hrs. MTBE (150mL) and water (150mL) were added and the aqueous layer further extracted with MTBE (2 x 100mL). The combined organics were washed with brine (100mL), dried (Na₂SO₄), and concentrated to give the intermediate 2,3-dimethyl-[1,4]benzoquinone as an orange-yellow solid (12.0g, 88%), which was used in the next step without further purification.

A solution of silver(I) nitrate (3.40g, 20 mmol) in water (50mL) was added to a mixture of 2,3-dimethyl-[1,4]benzoquinone (1.36g, 10 mmol) and 4-methylpentanoic acid (1.26mL, 1.16g, 20mmol) in acetonitrile (50mL) at ambient temperature. The mixture was stirred vigorously, heated to 75-80°C, and a solution of ammonium persulfate (4.56g, 20mmol) in water (30mL) added dropwise via syringe-pump over 4 hrs. After a total 20 hrs the majority of the acetonitrile was removed using a rotorevaporator, the residue partitioned between MTBE (100mL) and water (100mL), and the aqueous layer further extracted with MTBE (50mL). The combined organics were washed with 1:1 saturated brine-water (50mL) and then concentrated. The orange-red residue was purified by column chomratography on silica-gel using a gradient elution of 1 to 2.5% EtOAc-hexanes to give the 2,3-Dimethyl-5,6-bis-(3-methyl-butyl)-[1,4]benzoquinone as a yellow oil (200 mg, 7%). ¹H-NMR (CDCl₃, 400 MHz, δ ppm): 2.42-2.38 (4H, m), 1.99 (6H, s), 1.62 (2H, nonet, *J* = 7 Hz), 1.28-1.22 (4H, m), 0.93 (12H, d, *J* = 7 Hz). ¹³C-NMR (CDCl₃, 100 MHz, δ ppm): 187.60, 144.35, 140.39, 38.66, 28.71, 24.55, 22.35, 12.28.

### 2-(3-Hydroxy-3-methyl-butyl)-5,6-dimethyl-3-(3-methyl-but-2-enyl)-[1,4]benzoquinone (not of the invention)

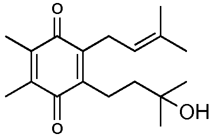

To a stirring solution of 113 mg 2,2,7,8-tetramethyl-5-(3-methyl-but-2-enyl)-chroman-6-ol (prepared in the method of Walkinshaw, et al., US 2005/0065099 A1, Mar. 24, 2005) in 3.75 mL acetonitrile-water (5:1) at 5 °C was added a yellow solution of cerium(IV) ammonium nitrate (475 mg) in acetonitrile-water (1:4, 2.75 mL) over a period of 5 minutes. The reaction mixture was allowed to stir for an additional 5 minutes, after which it was poured into a separatory funnel containing dichloromethane (30 mL) and water (30 mL). The aqueous layer was removed and the remaining organics were washed once with 1.0 M sodium chloride solution (30 mL). The organics were subsequently dried over anhydrous sodium sulfate, filtered, and concentrated in vacuo. Silica gel chromatography (15% ethyl acetate-85% hexanes) provided 58 mg of 2-(3-hydroxy-3-methyl-butyl)-5,6-dimethyl-3-(3-methyl-but-2-enyl)-[1,4]benzoquinone. ¹H NMR (CDCl₃, 400 MHz) 4.92 (*t*, 1H), 3.18 (*d*, 2H), 2.54 (*t*, 2H), 1.99 (*s,* 6H), 1.74 (*s,* 3H), 1.66 (*s,* 3H), 1.52 (*m*, 2H), 1.26 (*s,* 6H).

### 2-(3-Hydroxy-3-methyl-butyl)-5,6-dimethyl-3-(3-methyl-butyl)-[1,4]benzoquinone (not of the invention)

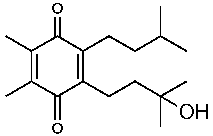

To a solution of 2,2,7,8-tetramethyl-5-(3-methyl-but-2-enyl)-chroman-6-ol (68 mg) in ethyl acetate (2.4 mL) was added Pd/C (26 mg, 5% w/w). The resulting suspension was flushed with hydrogen gas, the container sealed, and the contents stirred under 1 atm of hydrogen gas for 30 min. The mixture was then filtered and concentrated in vacuo. The resulting residue was dissolved in acetonitrile-water (5:1, 2.6 mL) and the solution cooled to 5 °C in an ice-water bath. Into the reaction mixture was added a solution of cerium(IV) ammonium nitrate (287 mg) in acetonitrile-water (1:4, 1.6 mL) over a period of 5 minutes. The reaction mixture was allowed to stir for an additional 5 minutes, after which it was poured into a separatory funnel containing dichloromethane (30 mL) and water (30 mL). The aqueous layer was removed and the remaining organics were washed once with 1.0 M sodium chloride solution (30 mL). The organics were subsequently dried over anhydrous sodium sulfate, filtered, and concentrated in vacuo. Silica gel chromatography (18% ethyl acetate-82% hexanes) provided 29 mg of 2-(3-hydroxy-3-methyl-butyl)-5,6-dimethyl-3-(3-methylbutyl)-[1,4]benzoquinone. ¹H NMR (CDCl₃, 400 MHz) 2.53 (*m,* 2H), 2.43 (*m,* 2H), 1.99 (*s*, 3H), 1.62 (*m*, 1H), 1.53 (*m*, 2H), 1.24 *(m,* 8H), 0.94 (*s*, 3H), 0.92 *(s,* 3H).

### (E)-1-(7-chloro-3-methylhept-2-enyl)-2,5-dimethoxy-3,4,6-trimethylbenzene (not of the invention)

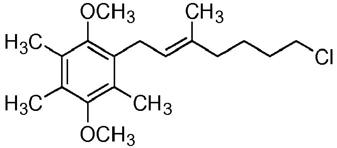

Zirconocene dichloride (1.66 g, 5.6 mmol) was treated with dichloroethane (22 mL) and trimethylaluminum solution (23 mmol, 2.0 M in heptane) was added. The clear yellow solution was stirred for 0.75 h and cooled to 0 °C. 5-chloro-1-pentyne was added neat over 1 h at 0 °C and stirred 10 h at room temperature. The clear brown solution was concentrated *in vacuo* and triturated with anhydrous hexanes (3 x 5 mL), the solvent being removed after each iteration via vacuum. Hexanes (10 mL) was then added and the solution cannulated away from the solids. The solids were rinsed with hexanes (5 mL) and the combined hexane solutions diluted with THF (80 mL). A prepared solution of 1-(chloromethyl)-2,5-dimethoxy-3,4,6-trimethylbenzene (4.009 g, 17.5 mmol) in THF (10 mL) was added to the vinyl alane *via* cannula and the combined solution cooled to 10 °C. The catalyst was prepared by treating bis-(triphenylphosphine)nickel dichloride (579 mg, 0.87 mmol) in THF (5 mL) with *n-*BuLi (1.6 M in hexanes, 1.75 mmol). The blood-red clear catalyst solution was then added *via* cannula to the 10 °C vinyl alane solution and let warm to room temperature over 7 h. The reaction was cooled to 0 °C and quenched by slow addition of 2.5 M HCl (100 mL) over 0.5 h followed by hexanes (100 mL) and separation. The aqueous layer was extracted 2 x100 mL hexanes, 1 x 50 mL 50 % EtOAc/hexanes and the combined organics washed once with brine (100 mL) and dried over anhydrous Na₂SO₄ and concentrated to a brown oil. Multiple flash chromatography yielded 5.2 g of (*E*)-1-(7-chloro-3 -methylhept-2-enyl)-2,5-dimethoxy-3,4,6-trimethylbenzene as a clear oil (94.6%). ¹H NMR (400 MHz, CDCl₃) d 5.05 (t, J = 6.4 Hz, 1H), 3.63 (S, 6H), 3.49 (t, J = 6.4 Hz, 2H), 3.35 (d, J = 8.4 Hz, 1H), 2.16 (s, 9H), 1.98 (t, J = 7.6 Hz, 2H), 1.76 (s, 3H), 1.70 (m, 2H), 1.52 (m, 2H). ¹³C NMR (100 MHz, CDCl₃) d 153.0, 152.7, 134.4, 131.4, 128.3, 127.9, 127.6, 123.8, 60.9, 60.1, 45.0, 38.8, 32.2, 26.1, 25.1, 16.1, 12.8, 12.7, 12.2.

### (E)-2-(7-chloro-3-methylhept-2-enyl)-3,5,6-trimethylcyclohexa-2,5-diene-1,4-dione (not of the invention)

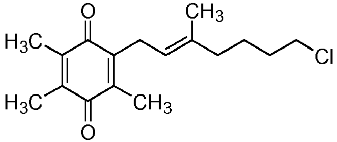

CAN (709 mg, 1.29 mmol) was dissolved into AcCN (7 mL) and water (3 mL) then cooled to 0 °C. In a separate flask, (*E*)-1-(7-Chloro-3-methylhept-2-enyl)-2,5-dimethoxy-3,4,6-trimethylbenzene (175 mg, 0.53 mmol) was dissolved into AcCN (2 mL) and 1 drop of H₂O and transferred to the stirring CAN solution. Stirring was maintained for 1 h, after which time an additional charge of CAN (350 mg) was added and let stir for 1 h. Water (10 mL) and EtOAc (10 mL) were added, the layers separated and the combined organics washed H₂O (2 x 5 mL). The combined aqueous phases were back extracted using EtOAc (2 x 5 mL) and the combined organics washed with brine (2 x 5 mL), dried over anhydrous Na₂SO₄ and concentrated to a yellow oil. Flash chromatography (SiO₂) yielded 29.7 mg of (*E*)-2-(7-chloro-3-methylhept-2-enyl)-3,5,6-trimethylcyclohexa-2,5-diene-1,4-dione as a yellow oil (18.7%). ¹H NMR (400 MHz, CDCl₃) δ□4.95 (t, *J* = *6.8 Hz,* 1H), 3.50 (t, *J* = *6.8 Hz,* 2H), 3.19 (d, *J* = *6.8 Hz,* 2H), 2.00 (m, 11H), 1.71 (m, 5H), 1.51 *(m, J* = *7.2 Hz,* 2H). ¹³C NMR (100 MHz, δ) 187.9, 187.0, 143.0, 140.4, 140.3, 136.5, 120.0, 44.9, 38.8, 32.1, 25.6, 24.9, 16.1, 12.3, 12.2.

### (E)-1-(6-chloro-3-methylhex-2-enyl)-2,5-dimethoxy-3,4,6-trimethylbenzene (not of the invention)

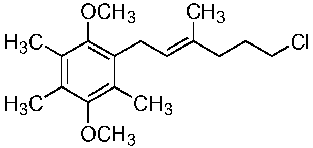

Zirconocene dichloride (982 mg, 3.36 mmol) was treated with trimethylaluminum (13.1 mL, 2.0 M in heptane) and dichloroethane (13 mL). The bright yellow solution was cooled to 0 °C and 5-chloro-1-pentyne added over 5 minutes. The reaction was held at 0 °C for 0.25 h and warmed to room temperature. After 5.5 h the dark yellow solution was reduced in vacuo to ca. 70% of its original volume and triturated with hexanes (2 x 10 mL). A final portion of hexanes (10 mL) was added and cannulated away from the precipitated salts with additional hexane wash (2 x 2 mL) to ensure complete transfer. The vinyl alane which was subsequently diluted with THF (40 mL) and treated with 1-(chloromethyl)-2,5-dimethoxy-3,4,6-trimethylbenzene (1.35 g) in THF (15 mL) via cannula. A separate flask containing bis-(triphenylphosphine)nickel dichloride (967 mg, 1.47 mmol) in THF (5 mL) was treated with n-buLi (260 mL, 1.6 M in heptane, 0.416 mmol) to give a dark red, clear solution which was added to the vinyl alane solution. The reaction was placed in a 15 °C water bath to control an exotherm and let stir overnight at room temperature. The reaction was quenched by treatment with citric acid (11 g) in H₂O (50 mL) via slow addition, followed by addition of hexanes (50 mL) and H₂O (50 mL) with stirring for an additional 20 minutes. The layers were separated and the aqueous phase extracted using hexanes (3 x 50 mL) then MTBE (2 x 50 mL). The combined organics were washed with brine (2 x 20 mL), dried over anhydrous Na₂SO₄ and concentrated to a brown oil. Flash chromatography yielded 2.011 g (74.0%) of (*E*)-1-(6-chloro-3-methylhex-2-enyl)-2,5-dimethoxy-3,4,6-trimethylbenzene as a clear oil. ¹H NMR (400 MHz, CDCl₃) d 5.11 (t, *J* = *7.2 Hz,* 1H), 3.65 (s, 6H), 3.48 (t, *J* = *6.8 Hz,* 2H), 3.76 (d, *J* = *6.4 Hz*, 2H), 2.18 (s, 9H), 2.10 (t, *J* = *7.2 Hz*, 2H) 1.84 (quintet, *J* = *7.2 Hz, 2H),* 1.78(s, 3H). ¹³C NMR (100 MHz, CDCl₃) d 153.0, 152.6, 133.4, 131.3,128.4, 127.9, 127.6, 124.5, 60.9, 60.1, 44.6, 36.6, 30.8, 26.1, 16.2, 12.8, 12.7, 12.2.

### 1-((E)-6-iodo-3-methylhex-2-enyl)-2,5-dimethoxy-3,4,6-trimethylbenzene (not of the invention)

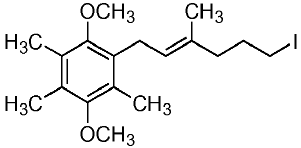

(*E*)-1-(6-chloro-3-methylhex-2-enyl)-2,5-dimethoxy-3,4,6-trimethylbenzene (725.8 mg, 2.334 mmol) and NaI (3.09 g, 20.61 mmol) were dissolved into acetone (10 mL) and heated to reflux for 18 h. The reaction mixture was cooled to room temperature and the cloudy solution added to H₂O (50 mL) and of a 50% EtOAc/hexanes solution (50 mL), the layers separated and the aqueous phase extracted with hexanes (3 x 25 mL), then MTBE (2 x 25 mL). The organics were combined and washed with saturated NaCl solution (2 x 25 mL) and dried over anhydrous Na₂SO₄. Concentration yielded 930.0 mg (99.0%) of 1-((*E*)-6-iodo-3-methylhex-2-enyl)-2,5-dimethoxy-3,4,6-trimethylbenzene as a pale yellow oil. ¹H NMR (400 MHz, CDCl₃) d 5.12 (t, J = 6.4 Hz, 1H), 3.65 (s, 6H), 3.36 (d, J = 6.0 Hz, 2H), 3.12 (t, J = 7.2 Hz, 2H), 2.18 (S 9H), 2.07 (t, J = 7.2 Hz, 2H), 1.91 (t, J = 7.2 HZ, 2H), 1.77 (s, 3H). ¹³C NMR (100 MHz, CDCl₃) d 153.1, 152.6, 133.0, 131.3, 128.4, 127.9, 127.6, 124.7, 60.9, 60.1, 40.1, 31.7, 26.1, 16.1, 12.8, 12.7, 12.2.

### (E)-2,3,5-trimethyl-6-(3-methylnon-2-enyl)-1,4-benzoquinone

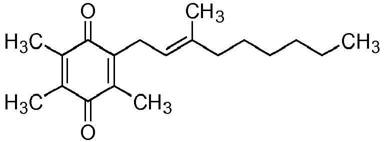

Zirconocene dichloride (220 mg, 0.755 mmol) was treated with trimethylaluminum in heptane (3 mL 2.0 M) and the solvent removed *in vacuo.* Dichloroethane (3 mL) was added and the yellow solution cooled to 0 °C prior to slow addition of 450 µL 1-octyne (336 mg, 3.05 mmol). The ice bath was removed after 20 minutes and the reaction warmed to rt over 2.5 h at which time it was concentrated *in vacuo* to a yellow slurry and triturated with hexanes (4 mL) and the solvent removed *in vacuo.*

Hexanes (3 mL) was added and the liquid cannulated away from the white solids. The solids were washed with 2 mL hexanes and the washings combined, concentrated to a yellow oil, dissolved into THF (5 mL) and cooled to -78 °C. 2-(chloromethyl)-3,5,6-trimethyl-1,4-benzoquinone (400 mg, 2.01 mmol) was dissolved into THF (3 mL) and transferred to the vinyl alane via cannula with THF (2 x 1 mL) to assist the transfer. *bis*-(Triphenylphosphine)nickel dichloride (66.2 mg, 0.101 mmol) was suspended in THF (2 mL) and treated with *n*-BuLi in hexanes (1.6 M, 0.20 mmol) to give a clear, blood red solution which was added via cannula to the chilled vinyl alane, chloromethyl quinone solution. The reaction was warmed to room temperature overnight and chilled to -20 C prior to addition of a 1 M citric acid solution (20 mL). The solution was stirred for 0.75 h, EtOAc (10 mL) added and the layers separated. The aqueous phase was extracted EtOAc (2 x 10 mL), the combined organics washed with brine (2 x 10 mL), dried over anhydrous Na₂SO₄ and concentrated to a yellow oil. Multiple flash chromatography yielded 138.7 mg (23.9%) of (E)-2,3,5-trimethyl-6-(3-methylnon-2-enyl)-1,4-benzoquinone as a yellow oil. ¹H NMR (400 MHz, CDCl₃) δ□4.91 (t, *J* = *6.8 Hz,* 1H), 3.18 (d, *J* = *6.8 Hz,* 2H), 2.00 (s, 9H), 1.92 (t, *J* = *8.0 Hz,* 2H), 1.70 (s, 3H), 1.34-1.21 (m, 8H), 0.84 (t, *J* = *6.8 Hz,* 3H). ¹³C NMR (100 MHz, CDCl₃) δ 187.8, 187.0, 143.2, 140.2, 137.4, 119.1, 39.6, 31.6, 28.8, 27.7, 25.5, 22.6, 16.1, 14.0, 12.3, 12.1

### (E)-7-(2,5-dimethoxy-3,4,6-trimethylphenyl)-5-methylhept-5-enenitrile (not of the invention)

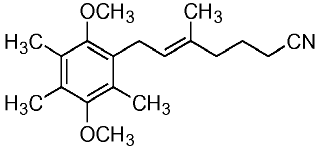

1-((*E*)-6-iodo-3-methylhex-2-enyl)-2,5-dimethoxy-3,4,6-trimethylbenzene (412 mg, 1.024 mmol) was combined with NaCN (247.7 mg) and dissolved in DMF (2 mL). The reaction was stirred for 25 h at 45 °C then cooled to room temperature. To the mixture was added H₂O (10 mL) followed by MTBE (6 mL) and the layers separated. The aqueous phase was extracted into MTBE (4 x 6 mL) and the combined organics washed with H₂O (2 x 5 mL) followed by saturated NaCl solution (2 x 5 mL) and dried over Na₂SO₄. The organics were concentrated to give (*E*)-7-(2,5-dimethoxy-3,4,6-trimethylphenyl)-5-methylhept-5-enenitrile as a pale yellow oil, 306.4 mg (99.0%). ¹H NMR (400 MHz, CDCl₃) d 5.14 (t, J = 6.4 Hz, 1H), 3.65 (s, 6H), 3.37 (d, J = 6.8 Hz, 2H), 2.26 (t, J = 7.2 Hz, 2H), 2.18 (s, 9H), 2.12 (t, J = 7.2 Hz, 2H), 1.76 (m, 4H).
¹³C NMR (100 MHz, CDCl₃) d 153.1, 152.6, 132.5, 131.0, 128.5, 128.0, 125.4, 119.7, 60.8, 60.1, 38.2, 38.2, 26.1, 23.5, 16.4, 15.9, 12.8, 12.7, 12.2.

### N-((E)-7-(2,5-dimethoxy-3,4,6-trimethylphenyl)-5-methylhept-5-enyl)acetamide (not of the invention)

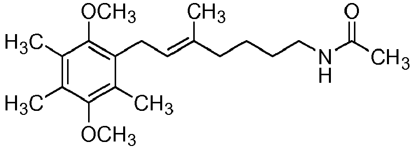

LAH (102 mg, 2.69 mmol) in THF (5 mL) was treated with (*E*)-7-(2,5-dimethoxy-3,4,6-trimethylphenyl)-5-methylhept-5-enenitrile (150.9 mg, 0.5006 mmol) in THF (5 mL) by dropping funnel over 10 minutes. After 4.25 h, the cloudy gray solution was placed in a room temperature water bath and treated carefully with Na₂SO₄•10H₂O (996 mg). The bath was removed and the reaction stirred vigorously for 1 h followed by an additional Na₂SO₄•10H₂O (959 mg) and stirring overnight. The white precipitate was separated from the organics, rinsed with EtOAc (5 x 5 mL) and concentrated to a clear, colorless oil of (*E*)-7-(2,5-dimethoxy-3,4,6-trimethylphenyl)-5-methylhept-5-en-1-amine.

The crude amine was dissolved into Et₃N (2 mL) and treated with neat Ac₂O (0.75 mL) over 5 minutes. The reaction exothermed slightly and was allowed to stir overnight at room temperature before being quenched by addition of H₂O (10 mL) and EtOAc (10 mL). The layers were separated and the aqueous phase extracted using EtOAc (3 x 10 mL). The combined organics were washed with H₂O (2 x 10 mL) and saturated NaCl solution (2 x 15 mL) before being dried over anhydrous Na₂SO₄ and concentration to a brown oil. Flash chromatography on silica gave N-((*E*)-7-(2,5-dimethoxy-3,4,6-trimethylphenyl)-5-methylhept-5-enyl)acetamide as an off-white crystalline solid, 130 mg (74.7%)

Data for (*E*)-7-(2,5-dimethoxy-3,4,6-trimethylphenyl)-5-methylhept-5-en-1-amine: ¹H NMR (400 MHz, CDCl₃) d 5.05 (t, J = 6.0 Hz, 1H), 3.63 (s, 6H), 3.35 (d, J = 6.0 Hz, 2H), 2.64 (t, J = 6.8 Hz, 2H), 2.16 (s, 9H), 1.97 (m, 2H), 1.75 (s, 3H), 1.63 (m, 2H), 1.38 (m, 3H). ¹³C NMR (100 MHz, CDCl₃) d 152.9,152.6,134.9,131.5,128.1, 127.8, 127.6, 123.2, 62.1, 60.7, 60.0, 42.0, 39.3, 33.4, 26.0, 16.1, 12.7, 12.6, 12.0. Data for N-((*E*)-7-(2,5-dimethoxy-3,4,6-trimethylphenyl)-5-methylhept-5-enyl)acetamide: ¹H NMR (400 MHz, CDCl₃) d 5.66 (br s, 1H), 5.04 (t, J = 6.0 Hz, 1H), 3.63 (s, 6H), 3.34 (d, J= 6.4 Hz, 2H), 3.18 (q, J = 5.6 Hz, 2H), 2.17 (s, 9H), 1.94 (m, 5H), 1.74 (S, 3H), 1.41 (m, 4H). ¹³C NMR (100 MHz, CDCl₃) d 169.9, 153.0, 152.6, 134.6, 131.4, 128.2, 127.8, 127.6, 123.5, 60.8, 60.0, 39.5, 39.1, 29.1, 26.1, 25.1, 23.2, 16.1, 12.7, 12.6, 12.1.

### N-((5E)-5-methyl-7-(2,4,5-trimethyl-3,6-dioxocyclohexa-1,4-dienyl)hept-5-enyl)acetamide (not of the invention)

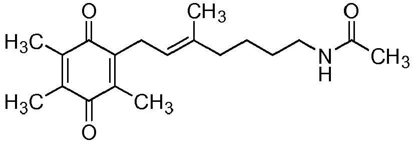

Ceric ammonium nitrate (90.1 mg, 0.164 mmol) was dissolved into H₂O (0.5 mL) and AcCN (0.5 mL) and cooled to 0 °C. A solution containing 25.2 mg N-((*E*)-7-(2,5-dimethoxy-3,4,6-trimethylphenyl)-5-methylhept-5-enyl)acetamide (0.0725 mmol) in AcCN (1 mL) and CH₂Cl₂ (0.25 mL) was added over 0.5 min. The reaction was stirred for 0.75 h at 0 °C then diluted with H₂O (2 mL). The layers were separated and the organic phase diluted with EtOAc (5 mL) and washed with H₂O (3 x 2 mL). The combined aqueous phase was back extracted using EtOAc (3 x 4 mL) and discarded. The combined organics were washed with brine (2 x 3 mL), dried over Na₂SO₄, concentrated in vacuo and subjected to flash chromatography (SiO₂) gave N-((*5E*)-5-methyl-7-(2,4,5-trimethyl-3,6-dioxocyclohexa-1,4-dienyl)hept-5-enyl)acetamide as a bright yellow liquid. ¹H NMR (400 MHz, CDCl₃) d 5.61 (br s, 1H), 3.20 (m, 4H), 2.01(m, 14H), 1.72(s, 3H), 1.41(m, 2H). ¹C NMR (100 MHz, CDCl₃) d 187.9, 187.1,170.0, 143.1, 140.43 140.37, 136.7, 119.9, 60.1, 39.5, 39.2, 29.1, 25.6, 25.0, 23.3, 16.1, 12.4, 12.2.

### (E)-7-(2,5-dimethoxy-3,4,6-trimethylphenyl)-5-methylhept-5-enal (not of the invention)

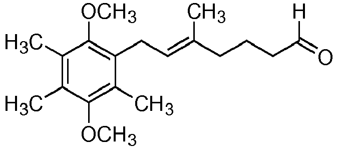

(*E*)-7-(2,5-dimethoxy-3,4,6-trimethylphenyl)-5-methylhept-5-enenitrile (178 mg, 0.59 mmol) was dried azeotropically with toluene in vacuo (3 x 2 mL), redissolved into toluene (3 mL) and cooled to 0 °C. DIBALH (1.0 M in heptane, 0.9 mmol) was added over 3 minutes dropwise. After 1 h, H₂O (2 ml) and aqueous H₂SO₄ (6 mL 2.5 M) were added and the mixture let warm to room temperature for 2.5 h. MTBE (5 mL) was added, the layers separated and the aqueous phase extracted 3 x 5 mL MTBE. The combined organics were washed with brine (10 mL), dried over anhydrous Na₂SO₄ and concentrated to give (*E*)-7-(2,5-dimethoxy-3,4,6-trimethylphenyl)-5-methylhept-5-enal as a colorless oil.

### (E)-7-(2,5-dimethoxy-3,4,6-trimethylphenyl)-5-methylhept-5-en-1-ol (not of the invention)

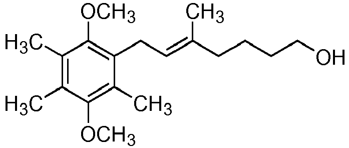

Crude (*E*)-7-(2,5-dimethoxy-3,4,6-trimethylphenyl)-5-methylhept-5-enenitrile in MeOH (3 mL) was cooled to 0 °C and treated with NaBH₄ (64.3 mg, 1.74 mmol), which gave immediate effervescence. After 12 h, H₂O (10 mL) was added (caution: copious gas evolution), MTBE (10 mL) was added, separated and the aqueous phase extracted with MTBE (3 x 10 mL). The combined organics were washed with H₂O (10 mL), brine (10 mL), dried over Na₂SO₄ and concentrated to give (E)-7-(2,5-dimethoxy-3,4,6-trimethylphenyl)-5-methylhept-5-en-1-ol as a pale yellow oil.

### (E)-7-(2,5-dimethoxy-3,4,6-trimethylphenyl)-5-methylhept-5-enyl acetate (not of the invention)

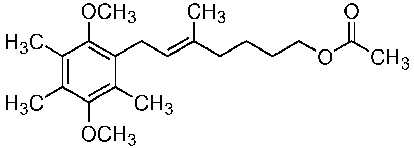

(E)-7-(2,5-dimethoxy-3,4,6-trimethylphenyl)-5-methylhept-5-en-1-ol in pyridine (2 mL) was treated with Ac₂O (2 mL) at 0 °C and let stir overnight. The reaction was quenched with H₂O (10 mL) followed by addition of EtOAc (10 mL) and separated. The aqueous phase was extracted with EtOAc (3 x 10 mL) and the combined organics washed with brine (15 mL), dried over anhydrous Na₂SO₄ and concentrated to a yellow oil. Flash chromatography on silica yielded 77.9 mg (62.3 %) of (*E*)-7-(2,5-dimethoxy-3,4,6-trimethylphenyl)-5-methylhept-5-enyl acetate as a clear oil.

### (E)-5-methyl-7-(2,4,5-trimethyl-3,6-dioxocyclohexa-1,4-dienyl)hept-5-enyl acetate (not of the invention)

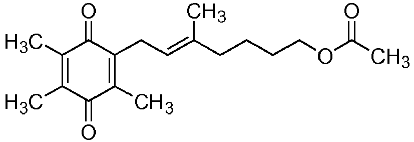

Ceric ammonium nitrate (270 mg, 0.498 mmol) was dissolved into H₂O (0.75 mL) and AcCN (1.5 mL) and the solution chilled to 0 °C. 77.9 mg of (*E*)-7-(2,5-dimethoxy-3,4,6-trimethylphenyl)-5-methylhept-5-enyl acetate (0.223 mmol) in AcCN (1.5 mL) was added over 2 minutes and the dark orange solution stirred for 0.5 h. H₂O (3 mL) and EtOAc (3 mL) were then added, the layers separated and the organics washed 2 x 2 mL H₂O. The combined aqueous phase was back extracted 3 x 5 mL EtOAc and the combined organics washed 2 x 5 mL saturated NaCl solution and dried over anhydrous Na₂SO₄. The resulting yellow liquid was concentrated to a yellow oil and subjected to flash chromatography, which yielded 25.8 mg of (*E*)-5-methyl-7-(2,4,5-trimethyl-3,6-dioxocyclohexa-1,4-dienyl)hept-5-enyl acetate as a bright yellow oil (36.2%). ¹H NMR (400 MHz, CDCl₃) d 4.95 (t, J = 6.4 Hz, 1H), 4.02 (t, J = 6.8 Hz, 2H), 3.19 (d, J = 6.8 Hz, 2H), 2.01 (m, 11H), 1.73 (s, 3H), 1.55 (m, 2H) 1.42 (m, 2H). ¹³C NMR (100 MHz, CDCl₃) d 187.9, 187.0, 171.1, 143.0, 140.4, 140.3, 136.7, 119.9, 64.4, 39.1, 28.1, 25.6, 24.1, 20.9, 16.1, 12.3, 12.1.

### (E)-2-(7-hydroxy-3-methylhept-2-enyl)-3,5,6-trimethylcyclohexa-2,5-diene-1,4-dione (not of the invention)

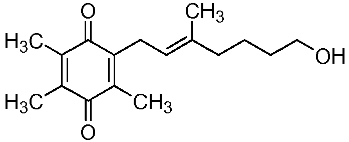

(*E*)-7-(2,5-dimethoxy-3,4,6-trimethylphenyl)-5-methylhept-5-en-1-ol (29.7 mg, 0.097 mmol) in AcCN (0.5 mL) with 2 drops of H₂O was cooled to 0 °C. CAN (114.7 mg, 0.209 mmol) was dissolved into AcCN (0.2 mL) and H₂O (0.5 mL) and added to a stirred solution of alcohol at 0 °C. The reaction was stirred at 0 °C for 1 h and H₂O (2 mL) and EtOAc (2 mL) was added, the layers separated and the aqueous phase extracted 3 x 2 mL EtOAc. The combined organics were washed 2 x 2 mL saturated brine, dried over anhydrous Na₂SO₄ and concentrated to a yellow oil. Flash chromatography yielded (*E*)-2-(7-hydroxy-3-methylhept-2-enyl)-3,5,6-trimethylcyclohexa-2,5-diene-1,4-dione (2.2 mg) as a yellow oil (8.2%). ¹³C NMR (100 MHz, CDCl₃) δ 187.9, 187.0, 143.1, 140.4, 104.3, 134.0, 119.7, 77.2, 62.9, 39.3, 32.3, 25.6, 24.0, 16.2, 12.4, 12.3, 12.2

Although the foregoing invention has been described in some detail by way of illustration and example for purposes of clarity of understanding, it is apparent to those skilled in the art that certain minor changes and modifications will be practiced. Therefore, the description and examples should not be construed as limiting the scope of the invention.

## Claims

1. A compound for use in a method of treating a mitochondrial disorder, wherein the compound is of the formula: 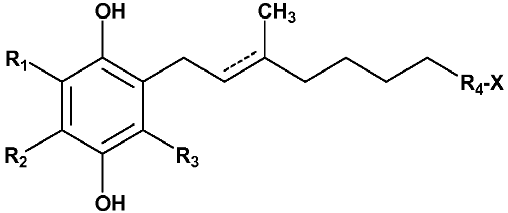 or 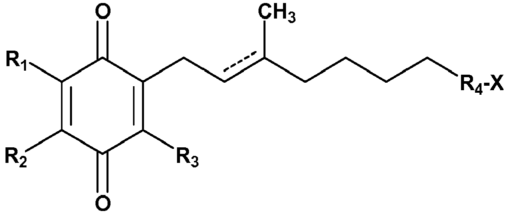
wherein the bond indicated with a dashed line is single or double;
where R₁, R₂, and R₃ are independently selected from the group consisting of -H and -C₁-C₅ alkyl;
where R₄ represents a linear or branched group containing 1 to 32 carbon atoms and any number of single, double, or triple bonds in any chemically possible combination;
where X is -H; and
where alkyl refers to a straight-chain, branched-chain, or cyclic saturated aliphatic group, and combinations thereof, having the number of carbon atoms specified; wherein the cyclic group consists of one ring or multiple fused rings;
or any stereoisomer, mixture of stereoisomers, or salt thereof;
wherein the mitochondrial disorder is selected from the group consisting of inherited mitochondrial diseases; Myoclonic Epilepsy with Ragged Red Fibers (MERRF); Mitochondrial Myopathy, Encephalopathy, Lactacidosis, Stroke (MELAS); Leber's Hereditary Optic Neuropathy (LHON); Leigh Syndrome; Kearns-Sayre Syndrome (KSS); Friedreich's Ataxia (FA); myopathies; cardiomyopathy; encephalomyopathy; renal tubular acidosis; neurodegenerative diseases; Parkinson's disease; Alzheimer's disease; amyotrophic lateral sclerosis (ALS); motor neuron diseases; neurological diseases; epilepsy; genetic diseases; Huntington's Disease; mood disorders; schizophrenia; bipolar disorder; age-associated diseases; macular degeneration; diabetes; and cancer.

2. The compound of claim 1 for use as defined in said claim, wherein R₁, R₂, and R₃ are independently -C₁-C₅ alkyl.

3. The compound of claim 2 for use as defined in said claim, wherein R₁ and R₂ are
-CH₃;
R₄ is -CH₂CH₂-; and
X is -H.

4. The compound of claim 3 for use as defined in said claim, which is of the formula: 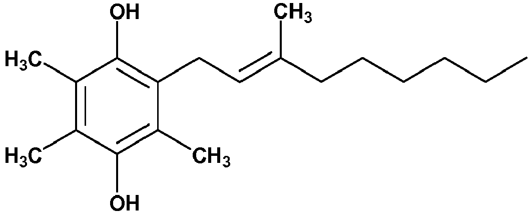 or 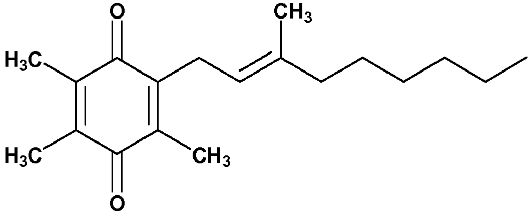 or 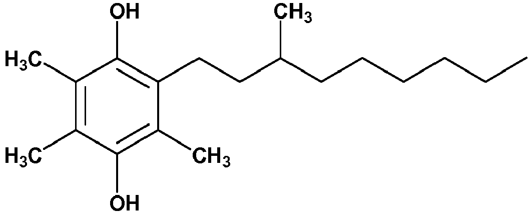 or 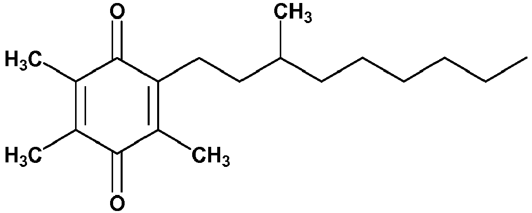 or 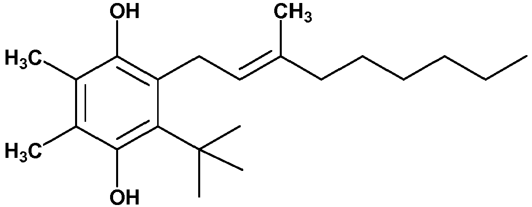 or 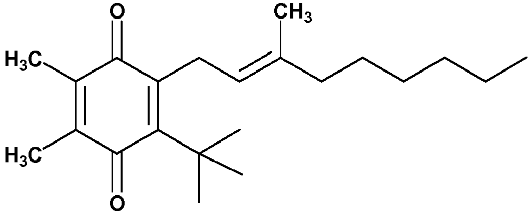 or any stereoisomer, or mixture of stereoisomers thereof.

5. The compound of claim 2 for use as defined in said claim, wherein R₄ is -(CH₂)ₙC(CH₃)₂-; where n is an integer from 0 to 15 inclusive.

6. A compound of the formula: 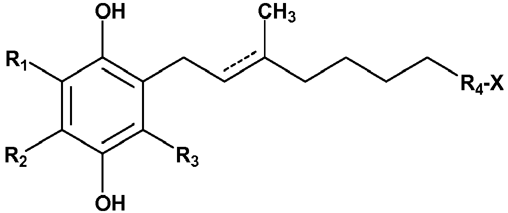 or 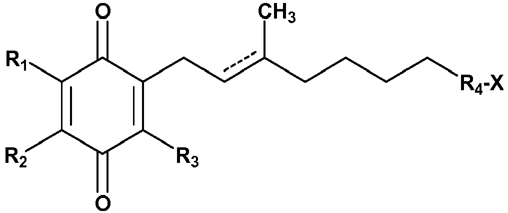 wherein the bond indicated with a dashed line is single or double;
where R₁, R₂, and R₃ are independently selected from the group consisting of -H and -C₁-C₅ alkyl, where at least one of R₁, R₂, and R₃ is independently selected from -C₂-C₅ alkyl;
where R₄ represents a linear or branched group containing 1 to 32 carbon atoms and any number of single, double, or triple bonds in any chemically possible combination;
where X is -H; and
where alkyl refers to a straight-chain, branched-chain, or cyclic saturated aliphatic group, and combinations thereof, having the number of carbon atoms specified; wherein the cyclic group consists of one ring or multiple fused rings;
or any stereoisomer, mixture of stereoisomers, or salt thereof.

7. A compound of the formula: 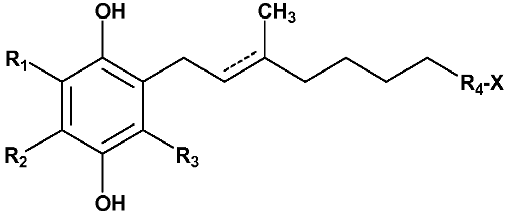 or 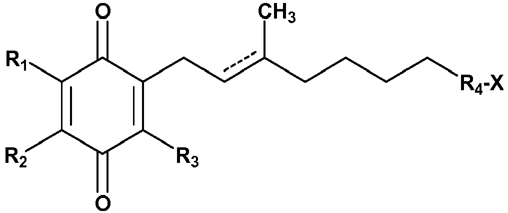
wherein the bond indicated with a dashed line is single or double;
where R₁, R₂, and R₃ are independently selected from the group consisting of -H and -C₁-C₅ alkyl;
where R₄ is -(CH₂)ₙC(CH₃)₂-, where n is an integer from 0 to 15 inclusive;
where X is -H; and
and
where alkyl refers to a straight-chain, branched-chain, or cyclic saturated aliphatic group, and combinations thereof, having the number of carbon atoms specified; wherein the cyclic group consists of one ring or multiple fused rings;
or any stereoisomer, mixture of stereoisomers, or salt thereof.

## Patentansprüche

1. Verbindung zur Verwendung in einem Verfahren des Behandelns einer mitochondrialen Störung, wobei die Verbindung die folgende Formel hat: 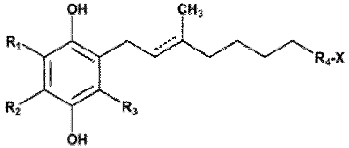 oder 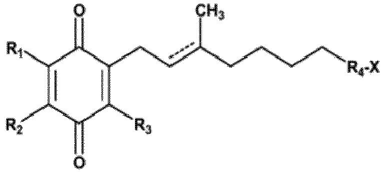
wobei die mit einer gestrichelten Linie angegebene Bindung einfach oder doppelt ist;
wobei R₁, R₂ und R₃ unabhängig aus der Gruppe bestehend aus -H und -C₁-C₅-Alkyl ausgewählt sind;
wobei R₄ eine lineare oder verzweigte Gruppe darstellt, die 1 bis 32 Kohlenstoffatome und eine beliebige Anzahl von Einfach-, Doppel- oder Dreifachbindungen in einer beliebigen chemisch möglichen Kombination enthält;
wobei X -H ist; und
wobei sich Alkyl auf eine geradkettige, verzweigtkettige oder cyclische gesättigte aliphatische Gruppe und Kombinationen davon bezieht, die die angegebene Anzahl Kohlenstoffatome haben; wobei die cyclische Gruppe aus einem Ring oder mehreren fusionierten Ringen besteht;
oder ein Stereoisomer, einem Mischung von Stereoisomeren oder ein Salz davon; wobei die mitochondriale Störung aus der Gruppe bestehend aus vererbten mitochondrialen Störungen; MERRF-Syndrom (Myoclonic Epilepsy with Ragged Red Fibers); mitochondrialer Myopathie, Enzephalopathie, Lactacidose, Schlaganfall (MELAS); Leberscher Optikusathrophie (Leber's Hereditary Optic Neuropathy, LHON); Leigh-Syndrom; Kearns-Sayre-Syndrom (KSS); Friedreich-Ataxie (FA) ; Myopathien; Kardiomyopathie; Enzephalopathie; renaler tubulärer Azidose; neurodegenerativen Erkrankungen; Morbus Parkinson; Morbus Alzheimer; amytropher Lateralsklerose (ALS); motorischen Neuronenerkrankungen; neurologischen Erkrankungen; Epilepsie; genetischen Krankheiten; Chorea Huntington; affektiven Störungen; Schizophrenie; bipolarer Störung; altersbedingten Krankheiten; Makuladegeneration; Diabetes; und Krebs ausgewählt ist.

2. Verbindung nach Anspruch 1 zur Verwendung wie in dem Anspruch definiert, wobei R₁, R₂ und R₃ unabhängig -C₁-C₅-Alkyl sind.

3. Verbindung nach Anspruch 2 zur Verwendung wie in dem Anspruch definiert, wobei R₁ und R₂ unabhängig -CH₃ sind;
R₄-CH₂CH₂- ist; und
X -H ist.

4. Verbindung nach Anspruch 3 zur Verwendung wie in dem Anspruch definiert, die die folgende Formel hat: 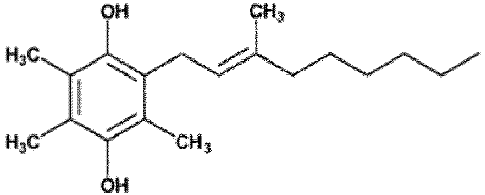 oder 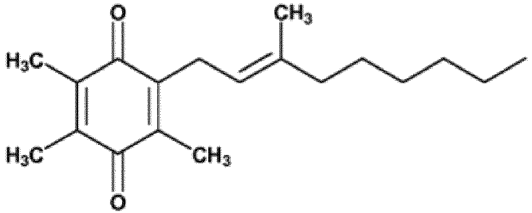 oder 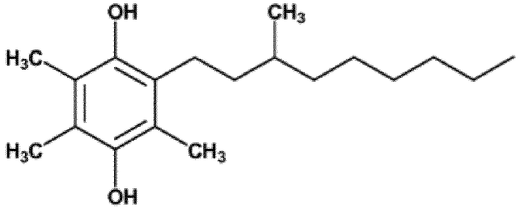 oder 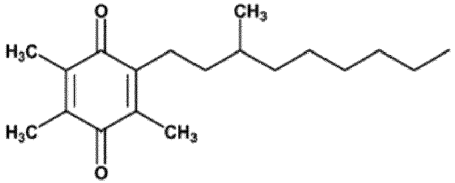 oder 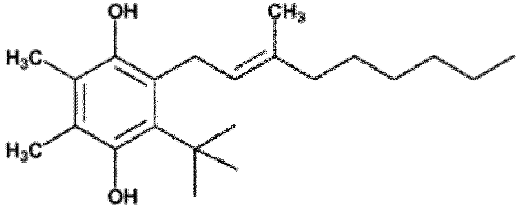 oder 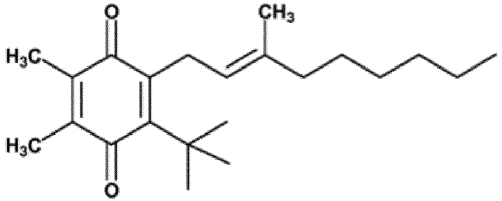 oder ein Stereoisomer oder eine Mischung von Stereoisomeren davon.

5. Verbindung nach Anspruch 2 zur Verwendung wie in dem Anspruch definiert, wobei R₄-(CH₂)ₙC(CH₃)₂- ist: wobei n eine Ganzzahl von 0 bis und mit 15 ist.

6. Verbindung der Formel: 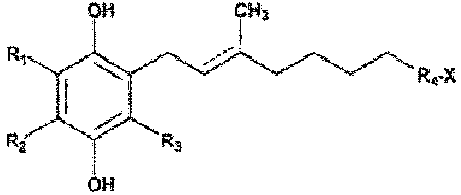 oder 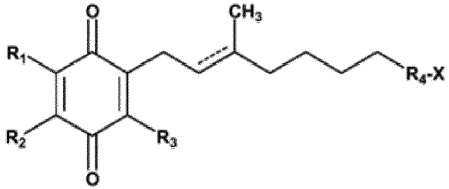 wobei die mit einer gestrichelten Linie angegebene Bindung einfach oder doppelt ist;
wobei R₁, R₂ und R₃ unabhängig aus der Gruppe bestehend aus -H und -C₁-C₅-Alkyl ausgewählt sind; wobei mindestens eines aus R₁, R₂ und R₃ unabhängig aus -C₂-C₅-Alkyl ausgewählt ist;
wobei R₄ eine lineare oder verzweigte Gruppe darstellt, die 1 bis 32 Kohlenstoffatome und eine beliebige Anzahl von Einfach-, Doppel- oder Dreifachbindungen in einer beliebigen chemisch möglichen Kombination enthält;
wobei X -H ist; und
wobei sich Alkyl auf eine geradkettige, verzweigtkettige oder cyclische gesättigte aliphatische Gruppe und Kombinationen davon bezieht, die die angegebene Anzahl Kohlenstoffatome haben; wobei die cyclische Gruppe aus einem Ring oder mehreren fusionierten Ringen besteht;
oder ein Stereoisomer, eine Mischung von Stereoisomeren oder ein Salz davon.

7. Verbindung der Formel: 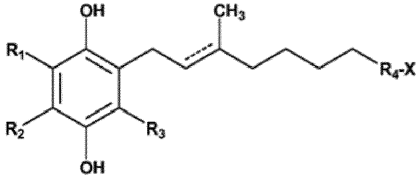 oder 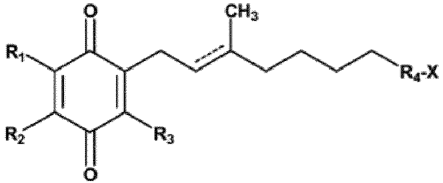
wobei die mit einer gestrichelten Linie angegebene Bindung einfach oder doppelt ist;
wobei R₁, R₂ und R₃ unabhängig aus der Gruppe bestehend aus -H und -C₁-C₅-Alkyl ausgewählt sind;
wobei R₄ -(CH₂)ₙC(CH₃)₂- ist, wobei n eine Ganzzahl von 0 bis und mit 15 ist;
wobei X -H ist; und
und
wobei sich Alkyl auf eine geradkettige, verzweigtkettige oder cyclische gesättigte aliphatische Gruppe und Kombinationen davon bezieht, die die angegebene Anzahl Kohlenstoffatome haben; wobei die cyclische Gruppe aus einem Ring oder mehreren fusionierten Ringen besteht;
oder ein Stereoisomer, eine Mischung von Stereoisomeren oder ein Salz davon.

## Revendications

1. Composé pour une utilisation dans un procédé de traitement d'un trouble mitochondrial, le composé étant de formule : 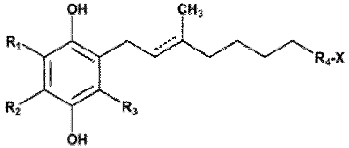 ou 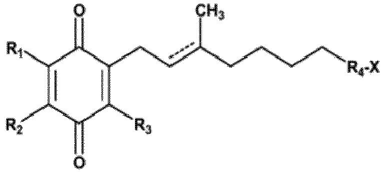
dans laquelle la liaison indiquée avec un trait pointillé est simple ou double ;
où R₁, R₂ et R₃ sont indépendamment choisis dans le groupe constitué par -H et un groupe alkyle en C₁-C₅ ;
où R₄ représente un groupe linéaire ou ramifié contenant 1 à 32 atomes de carbone et un nombre quelconque des liaisons simples, doubles ou triples dans une quelconque combinaison chimiquement possible ;
où X est -H ; et
où alkyle fait référence à un groupe aliphatique saturé à chaîne droite, à chaîne ramifiée ou cyclique, et des combinaisons de celui-ci, ayant le nombre d'atomes de carbone spécifié ; le groupe cyclique étant constitué d'un cycle ou de plusieurs cycles fusionnés ;
ou tout stéréoisomère, mélange de stéréoisomères ou sel de celui-ci ; le trouble mitochondrial étant choisi dans le groupe constitué par des maladies mitochondriales héréditaires ; l'épilepsie myoclonique avec fibres rouges déchiquetées (MERRF); la myopathie mitochondriale, une encéphalopathie, la lactacidose, l'AVC (MELAS) ; la neuropathie optique héréditaire de Leber (LHON) ; le syndrome de Leigh ; le syndrome de Kearns-Sayre (KSS) ; l'ataxie de Friedreich (FA) ; les myopathies ; la cardiomyopathie ; l'encéphalomyopathie ; l'acidose tubulaire rénale ; les maladies neurodégéneratives ; la maladie de Parkinson ; la maladie d'Alzheimer ; la sclérose latérale amyotrophique (ALS) ; les maladies neuro-motrices ; les maladies neurologiques ; l'épilepsie ; les maladies génétiques ; la chorée de Huntington ; les troubles de l'humeur ; la schizophrénie ; un trouble bipolaire ; les maladies liées à l'âge ; la dégénérescence maculaire ; le diabète ; et le cancer.

2. Composé de la revendication 1, pour une utilisation telle que définie dans ladite revendications, où R₁, R₂ et R₃ sont indépendamment un groupe alkyle en C₁-C₅.

3. Composé de la revendication 2 pour une utilisation telle que définie dans ladite revendication, dans lequel R₁ et R₂ sont -CH₃ ;
R₄ est - CH₂CH₂- ; et
X est -H.

4. Composé de la revendication 3 pour une utilisation telle que définie dans ladite revendication, qui est de formule 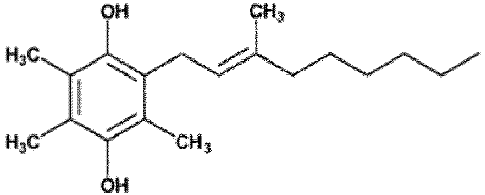 ou 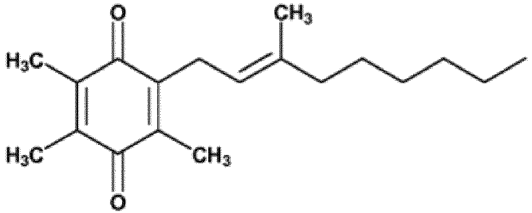 ou 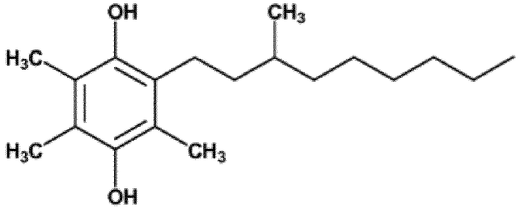 ou 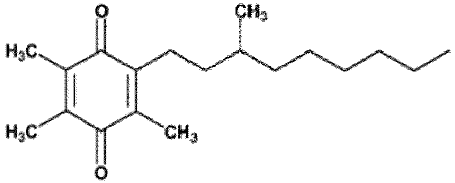 ou 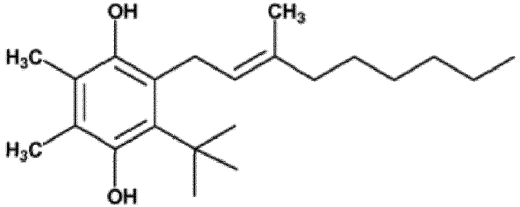 ou

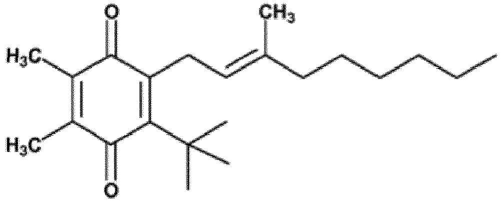

5. Composé de la revendication 2 pour une utilisation telle que définie dans ladite revendication, dans lequel R₄ est -(CH₂)ₙC(CH₃)₂- ; où n est un entier de 0 à 15 inclus.

6. Composé de formule 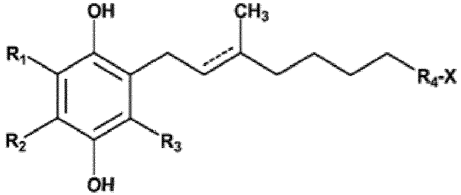 ou 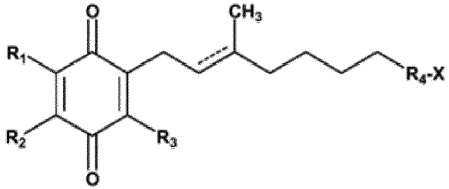 dans laquelle la liaison indiquée avec un trait pointillé est simple ou double ;
où R₁, R₂ et R₃ sont indépendamment choisis dans le groupe constitué par -H et un groupe alkyle en C₁-C₅, où au moins l'un de R₁, R₂ et R₃ est indépendamment choisi parmi un groupe alkyle en C₂-C₅ ;
où R₄ représente un groupe linéaire ou ramifié contenant 1 à 32 atomes de carbone et un nombre quelconque des liaisons simples, doubles ou triples dans une quelconque combinaison chimiquement possible ;
où X est -H ; et
où alkyle fait référence à un groupe aliphatique saturé à chaîne droite, à chaîne ramifiée ou cyclique, et des combinaisons de celui-ci, ayant le nombre d'atomes de carbone spécifié ; le groupe cyclique étant constitué d'un cycle ou de plusieurs cycles fusionnés ;
ou tout stéréoisomère, mélange de stéréoisomères ou sel de celui-ci.

7. Composé de formule : 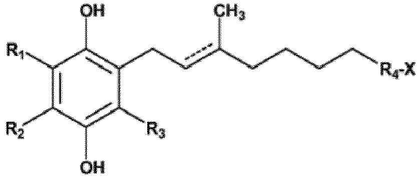 ou 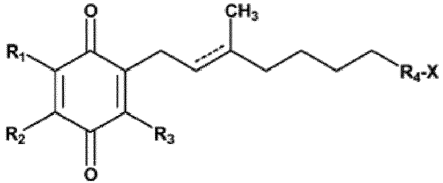
dans laquelle la liaison indiquée avec un trait pointillé est simple ou double ;
où R₁, R₂ et R₃ sont indépendamment choisis dans le groupe constitué par -H et un groupe alkyle en C₁-C₅ ;
où R₄ est -(CH₂)ₙC(CH₃)₂-, où n est un entier de 0 à 15 inclus ;
où X est -H ; et
et
où alkyle fait référence à un groupe aliphatique saturé à chaîne droite, à chaîne ramifiée ou cyclique, et des combinaisons de celui-ci, ayant le nombre d'atomes de carbone spécifié ; le groupe cyclique étant constitué d'un cycle ou de plusieurs cycles fusionnés ;
ou tout stéréoisomère, mélange de stéréoisomères ou sel de celui-ci.
